# EUROPEAN PATENT APPLICATION

(11) **EP 4 640 709 A1**
(43) Date of publication of application: **29.10.2025**
(21) Application number: 23907154.1
(22) Date of filing: 21.12.2023
(51) Int. Cl.: C07K 16/28, A61K 39/395, A61P 1/04, A61P 3/10, A61P 9/10, A61P 13/12, A61P 17/00, A61P 19/02, A61P 25/00, A61P 29/00, A61P 35/00, A61P 37/06, C12N 15/13, G01N 33/53

(54) **ANTI-HUMAN CX3CR1 ANTIBODY**

(30) Priority: 22.12.2022 JP 2022205800
(71) Applicant: CHIOME BIOSCIENCE INC., Shibuya-ku Tokyo 151-0071 (JP); National Center of Neurology and Psychiatry, Kodaira-shi Tokyo 187-8551 (JP)
(72) Inventor: SATO Yutaka, Tokyo 151-0071 (JP); YAMAMOTO Kotaro, Tokyo 151-0071 (JP); TAKESUE Aki, Tokyo 151-0071 (JP); SAKAGUCHI Izumi, Tokyo 151-0071 (JP); YAMAMURA Takashi, Kodaira-shi, Tokyo 187-8551 (JP); OKI Shinji, Kodaira-shi, Tokyo 187-8551 (JP); RAVENEY Benjamin Joseph Edward, Kodaira-shi, Tokyo 187-8551 (JP)
(74) Representative: J A Kemp LLP
(86) International application number: PCT/JP2023/046028
(87) International publication number: WO 2024/135794

(57) **Abstract**

The present invention aims to provide an anti-human CX3CR1 antibody, etc. The present invention is directed to an antibody against human CX3CR1 having particular CDR sequences or an antibody fragment thereof, etc.

## Description

### Technical Field

The present invention relates to an antibody against human CX3CR1 or a fragment thereof, and uses thereof.

### Background Art

CX3CR1 is a G protein-coupled receptor serving as a chemokine receptor. CX3CR1 is expressed on immunocytes including cytotoxic lymphocytes (e.g., NK cells, cytotoxic T cells), mature macrophages and mucosal dendritic cells, which are deeply involved in the elimination of pathogens or abnormal cells. The unique ligand of CX3CR1 is CX3CL1 (fractalkine (FKN)) whose expression is known to be induced when vascular endothelial cells are treated with inflammatory cytokines TNF and IL-1, and FKN is expressed on the surface of injured vascular endothelial cells and smooth muscle cells to mediate leukocyte adhesion. FKN is also released into the circulatory system through proteolytic cleavage and functions as a chemoattractant.

FKN is induced on vascular endothelial cells during inflammation, as described above, while CX3CR1 can be found on many types of leukocytes; and hence it has been strongly suggested that FKN and CX3CR1 are also involved in pathogenesis and progress in inflammatory diseases. For example, in autoimmune diseases including rheumatoid arthritis, inflammatory bowel disease, lupus nephritis and multiple sclerosis, it has been suggested that FKN and CX3CR1 are involved in their onset and pathology (Non-patent Document 1).

Moreover, in humans, less active CX3CR1 variants (V249I/T280M) have been shown to be related to the risks of cardiovascular diseases (Non-patent Document 2), coronary artery disease (angiographic evidence of stenosis) (Non-patent Document 3) and carotid artery occlusive disease (Non-patent Document 4).

As a therapeutic agent with anti-inflammatory action through suppression of the migration and/or infiltration of CX3CR1-expressing immunocytes caused by binding between FKN and CX3CR1 as described above, there has been developed an anti-FKN antibody (Patent Document 1) or an antibody analogue (Patent Document 2), etc., which exerts an effect through inhibition of binding between FKN and CX3CR1. Moreover, a possibility has been suggested that an anti-CX3CR1 antibody may exert an effect in the treatment of multiple sclerosis (Patent Document 3).

### Prior Art Documents

### Patent Documents

[Patent Document 1] WO2006/046739
[Patent Document 2] WO2013/130381
[Patent Document 3] WO2018/101261

### Non-patent Documents

[Non-patent Document 1] Umehara H, Blood Et, Okazaki T, et al.: Fractalkine in Vascular Biology: From Basic Research to Clinical Disease, Arterioscler Thomb Vasc Biol 24:34-40, 2004
[Non-patent Document 2] McDermott, 2001; Circ Res 89:401
[Non-patent Document 3] McDermott, 2003; J. Clin. Invest. 111: 1241
[Non-patent Document 4] Ghilardi, 2004; Stroke 35:1276

### Summary of the Invention

### Problem to be Solved by the Invention

However, it is only a few diseases for which a significant therapeutic effect has been demonstrated in these documents.

Under such circumstances, there has been a demand for further development of therapeutic agents for diseases in which CX3CR1-expressing immunocytes are involved. An object of the present invention is to provide a prophylactic agent, a progression suppressor, a therapeutic agent or the like which binds to human CX3CR1 and is targeted for diseases in which human CX3CR1-expressing immunocytes are involved, as exemplified by a prophylactic agent, a progression suppressor, a therapeutic agent or the like for progressive immune-mediated demyelinating diseases (preferably progressive immune-mediated demyelinating diseases of the central nervous system, e.g., secondary progressive multiple sclerosis (SPMS)).

### Means to Solve the Problem

The present invention has been made in consideration of the above situation and aims to provide an anti-CX3CR1 antibody and an antibody fragment thereof, as well as uses thereof (e.g., a pharmaceutical composition), etc., as shown below.
[1] An antibody against human CX3CR1, wherein
   the amino acid sequences of heavy chain variable region (VH) complementarity determining region (CDR) 1 (CDR-H1), CDR2 (CDR-H2) and CDR3 (CDR-H3) consist of:
   the amino acid sequences shown in SEQ ID NOs: 2, 3 and 4, respectively, and
   the amino acid sequences of light chain variable region (VL) CDR1 (CDR-L1), CDR2 (CDR-L2) and CDR3 (CDR-L3) consist of:
      the amino acid sequences shown in SEQ ID NOs: 6, 7 and 8, respectively; or
      the amino acid sequences shown in SEQ ID NOs: 10, 7 and 8, respectively.
[2] The antibody according to [1] above, wherein
   (a)
      the amino acid sequences of CDR-H1, CDR-H2 and CDR-H3 consist of the amino acid sequences shown in SEQ ID NOs: 2, 3 and 4, respectively, and
      the amino acid sequences of CDR-L1, CDR-L2 and CDR-L3 consist of the amino acid sequences shown in SEQ ID NOs: 6, 7 and 8, respectively, or
   (b)
      the amino acid sequences of CDR-H1, CDR-H2 and CDR-H3 consist of the amino acid sequences shown in SEQ ID NOs: 2, 3 and 4, respectively, and
      the amino acid sequences of CDR-L1, CDR-L2 and CDR-L3 consist of the amino acid sequences shown in SEQ ID NOs: 10, 7 and 8, respectively.
[3] An antibody against human CX3CR1, wherein
   the amino acid sequence of the heavy chain variable region (VH) consists of the amino acid sequence shown in SEQ ID NO: 1, 11, 12, 13 or 14, and
   the amino acid sequence of the light chain variable region (VL) consists of the amino acid sequence shown in SEQ ID NO: 5, 9 or 15.
[4] The antibody according to [3] above, wherein
   (a)
      the amino acid sequence of VH consists of the amino acid sequence shown in SEQ ID NO: 1, and
      the amino acid sequence of VL consists of the amino acid sequence shown in SEQ ID NO: 5,
   (b)
      the amino acid sequence of VH consists of the amino acid sequence shown in SEQ ID NO: 1, and
      the amino acid sequence of VL consists of the amino acid sequence shown in SEQ ID NO: 9,
   (c)
      the amino acid sequence of VH consists of the amino acid sequence shown in SEQ ID NO: 11, and
      the amino acid sequence of VL consists of the amino acid sequence shown in SEQ ID NO: 15,
   (d)
      the amino acid sequence of VH consists of the amino acid sequence shown in SEQ ID NO: 12, and
      the amino acid sequence of VL consists of the amino acid sequence shown in SEQ ID NO: 15,
   (e)
      the amino acid sequence of VH consists of the amino acid sequence shown in SEQ ID NO: 13, and
      the amino acid sequence of VL consists of the amino acid sequence shown in SEQ ID NO: 15, or
   (f)
      the amino acid sequence of VH consists of the amino acid sequence shown in SEQ ID NO: 14, and
      the amino acid sequence of VL consists of the amino acid sequence shown in SEQ ID NO: 15.
[5] The antibody according to any one of [1] to [4] above, wherein the antibody is a humanized antibody.
[6] The antibody according to any one of [1] to [4] above, which has inhibitory or suppressive activity against the interaction or binding between human CX3CR1 and human CX3CL1 (fractalkine).
[7] The antibody according to any one of [1] to [4] above, which has inhibitory or suppressive activity against the function of Eomes-expressing Th cells (helper T cells).
   The present invention also encompasses the antibody according to any one of [1] to [4] above, which has inhibitory or suppressive activity against the function of Granzyme B (GZMB)-expressing Th cells or Eomes- and Granzyme B-expressing Th cells.
[8] The antibody according to [7] above, which has inhibitory or suppressive activity against the infiltration of Eomes-expressing Th cells into the CNS (central nervous system).
   The present invention also encompasses the antibody according to any one of [1] to [4] above, which has inhibitory or suppressive activity against the infiltration of Granzyme B-expressing Th cells or Eomes- and Granzyme B-expressing Th cells into the CNS.
[9] The antibody according to any one of [1] to [4] above, which has the function of improving experimental autoimmune encephalomyelitis (EAE) pathology or suppressing the progression of EAE pathology.
[10] The antibody according to any one of [1] to [4] above, which is used for the prevention, suppression of progression, or treatment of a progressive immune-mediated demyelinating disease, preferably a progressive immune-mediated demyelinating disease of the central nervous system; an autoimmune disease, preferably rheumatoid arthritis, inflammatory bowel disease, Crohn's disease, systemic scleroderma, systemic lupus erythematosus, or autoimmune myositis; a heart disease including atherosclerosis; a chronic renal disease; a diabetic heart disease; a diabetic renal disease; an interstitial lung disease; a cancer, preferably breast cancer, prostate cancer, or colorectal cancer; a COVID-19-related central nervous system disease; or a neurodegenerative disease, preferably ALS, Alzheimer's disease, or multiple system atrophy.
[11] The antibody according to [10] above, wherein the progressive immune-mediated demyelinating disease is secondary progressive multiple sclerosis (SPMS).
[12] An antibody fragment derived from the antibody according to any one of [1] to [4] above.
[13] A pharmaceutical composition comprising the antibody according to any one of [1] to [4] above or the antibody fragment according to [12] above.
[14] The pharmaceutical composition according to [13] above, which is used for the prevention, suppression of progression, or treatment of a progressive immune-mediated demyelinating disease, preferably a progressive immune-mediated demyelinating disease of the central nervous system; an autoimmune disease, preferably rheumatoid arthritis, inflammatory bowel disease, Crohn's disease, systemic scleroderma, systemic lupus erythematosus, or autoimmune myositis; a heart disease including atherosclerosis; a chronic renal disease; a diabetic heart disease; a diabetic renal disease; an interstitial lung disease; a cancer, preferably breast cancer, prostate cancer, or colorectal cancer; a COVID-19-related central nervous system disease; or a neurodegenerative disease, preferably ALS, Alzheimer's disease, or multiple system atrophy.
[15] The pharmaceutical composition according to [14] above, wherein the progressive immune-mediated demyelinating disease is secondary progressive multiple sclerosis (SPMS).
[16] The pharmaceutical composition according to [13] above, which is used to inhibit or suppress the interaction or binding between human CX3CR1 and human CX3CL1 (fractalkine).
[17] The pharmaceutical composition according to [13] above, which is used to inhibit or suppress the function of Eomes-expressing Th cells.
   The present invention also encompasses the pharmaceutical composition according to [13] above, which is used to inhibit or suppress the function of Granzyme B-expressing Th cells or Eomes- and Granzyme B-expressing Th cells.
[18] The pharmaceutical composition according to [17] above, which is used to inhibit or suppress the infiltration of Eomes-expressing Th cells into the CNS.
   The present invention also encompasses the pharmaceutical composition according to [13] above, which is used to inhibit or suppress the infiltration of Granzyme B-expressing Th cells or Eomes- and Granzyme B-expressing Th cells into the CNS.
[19] The pharmaceutical composition according to [13] above, which is used to improve experimental autoimmune encephalomyelitis (EAE) pathology or suppress the progression of EAE pathology.
[20] A method for the prevention, suppression of progression, or treatment of a progressive immune-mediated demyelinating disease, preferably a progressive immune-mediated demyelinating disease of the central nervous system; an autoimmune disease, preferably rheumatoid arthritis, inflammatory bowel disease, Crohn's disease, systemic scleroderma, systemic lupus erythematosus, or autoimmune myositis; a heart disease including atherosclerosis; a chronic renal disease; a diabetic heart disease; a diabetic renal disease; an interstitial lung disease; a cancer, preferably breast cancer, prostate cancer, or colorectal cancer; a COVID-19-related central nervous system disease; or a neurodegenerative disease, preferably ALS, Alzheimer's disease, or multiple system atrophy, wherein the method comprises administering the pharmaceutical composition according to [13] above to a subject.
[21] The method according to [20] above, wherein the progressive immune-mediated demyelinating disease is secondary progressive multiple sclerosis (SPMS).
[22] A method for inhibiting or suppressing the interaction or binding between human CX3CR1 and human CX3CL1 (fractalkine), wherein the method comprises administering the pharmaceutical composition according to [13] above to a subject.
[23] A method for inhibiting or suppressing the function of Eomes-expressing Th cells, wherein the method comprises administering the pharmaceutical composition according to [13] above to a subject.
   The present invention also encompasses a method for inhibiting or suppressing the function of Granzyme B-expressing Th cells or Eomes- and Granzyme B-expressing Th cells, wherein the method comprises administering the pharmaceutical composition according to [13] above to a subject.
[24] The method according to [23] above, which is a method for inhibiting or suppressing the infiltration of Eomes-expressing Th cells into the CNS, wherein the method comprises administering the pharmaceutical composition according to [13] above to a subject.
   The present invention also encompasses a method for inhibiting or suppressing the infiltration of Granzyme B-expressing Th cells or Eomes- and Granzyme B-expressing Th cells into the CNS, wherein the method comprises administering the pharmaceutical composition according to [13] above to a subject.
[25] A method for improving experimental autoimmune encephalomyelitis (EAE) pathology or suppressing the progression of EAE pathology, wherein the method comprises administering the pharmaceutical composition according to [13] above to a subject.
[26] A kit for use in the prevention, suppression of progression, or treatment of, or for use in the diagnosis of a progressive immune-mediated demyelinating disease, preferably a progressive immune-mediated demyelinating disease of the central nervous system; an autoimmune disease, preferably rheumatoid arthritis, inflammatory bowel disease, Crohn's disease, systemic scleroderma, systemic lupus erythematosus, or autoimmune myositis; a heart disease including atherosclerosis; a chronic renal disease; a diabetic heart disease; a diabetic renal disease; an interstitial lung disease; a cancer, preferably breast cancer, prostate cancer, or colorectal cancer; a COVID-19-related central nervous system disease; or a neurodegenerative disease, preferably ALS, Alzheimer's disease, or multiple system atrophy, wherein the kit comprises the antibody according to any one of [1] to [4] above or the antibody fragment according to [12] above.
[27] The kit according to [26] above, wherein the progressive immune-mediated demyelinating disease is secondary progressive multiple sclerosis (SPMS).

### Effects of the Invention

The present invention enables the provision of an antibody binding to human CX3CR1, etc. The anti-human CX3CR1 antibody according to the present invention may have, for example, inhibitory or suppressive activity against the interaction or binding between human CX3CR1 and human CX3CL1 (fractalkine), or the function of improving experimental autoimmune encephalomyelitis (EAE) pathology or suppressing the progression of EAE pathology. Moreover, the anti-human CX3CR1 antibody according to the present invention may exert an effect as a prophylactic agent, a progression suppressor or a therapeutic agent for progressive immune-mediated demyelinating diseases (preferably progressive immune-mediated demyelinating diseases of the central nervous system, e.g., secondary progressive multiple sclerosis (SPMS)).

### Brief Description of the Drawings

Figure 1 shows the results measured by flow cytometry for the binding activity of the m1F8 antibody to human CX3CR1-expressing Ba/F3 cells.
Figure 2 shows the results measured for the inhibitory activity of the m1F8 antibody against the fractalkine-dependent cell migration of human CX3CR1-expressing Ba/F3 cells.
Figure 3 shows schematic views of chimeric CX3CR1 expression constructs prepared for epitope analysis, in which extracellular domains were exchanged between human and mouse CX3CR1. Panel (A) shows human CX3CR1-based expression constructs, and panel (B) shows mouse CX3CR1-based expression constructs.
Figure 4 shows the heavy and light chain variable region sequences of m1F8 antibody variants, i.e., m1F8-1 and m1F8-2 antibodies. The underlined regions in each sequence represent CDR sequences.
Figure 5 shows the test results obtained when anti-CX3CR1-Nanobody (BII00313) was administered to NR4A2/CX3CR1 genetically modified mice induced to develop monophasic EAE (a human CX3CR1-KI mouse late-onset EAE model). Panel (A) shows the blood level of free fractalkine (CX3CL1), and panel (B) shows EAE scores.
Figure 6 shows the test results obtained when the m1F8-1 or m1F8-2 antibody was administered to the human CX3CR1-KI mouse late-onset EAE model. Panel (A) shows the blood level of free fractalkine, and panel (B) shows EAE scores.
Figure 7 shows the EAE scores obtained in a test in which m1F8-2 or anti-CX3CR1-Nanobody (BII00313) was administered to the human CX3CR1-KI mouse late-onset EAE model.
Figure 8 shows the heavy and light chain variable region sequences of 4 types of humanized 1F8 antibodies (h1F8-2-1, h1F8-2-2, h1F8-2-3 and h1F8-2-4). The underlined regions in each sequence represent CDR sequences.
Figure 9 shows the results measured by flow cytometry for the binding activity of the humanized 1F8 antibody (h1F8-2-2) or 306D-hFc to human, cynomolgus monkey, mouse or rat CX3CR1-expressing Ba/F3 cells.
Figure 10 shows the results measured by flow cytometry for the binding activity of the h1F8-2-2 antibody or 306D-hFc to Ba/F3 cells expressing chimeric CX3CR1 in which extracellular domains were exchanged between human and mouse CX3CR1.
Figure 11 shows the results measured for the *in vitro* inhibitory activity of the h1F8-2-2 antibody or BII00313 in Ca-influx assay using human CX3CR1-expressing CHO-K1 cells. Panel (A) shows the results measured simultaneously for the inhibitory activity of the h1F8-2-2 antibody, BII00313 or Synagis, panel (B) shows the results measured for the concentration-dependent inhibitory activity of the h1F8-2-2 antibody, and panel (C) shows the results measured for the concentration-dependent inhibitory activity of BII00313.
Figure 12 shows the ratio of CD115 positive cells ((A): m1F8-2 staining, (B): 66B02-mFc staining), the ratio of CD115 and CX3CR1 double-positive cells ((C): m1F8-2 staining, (D): 66B02-mFc staining), and the ratio of CX3CR1 positive cells relative to CD115 positive cells ((E): m1F8-2 staining, (F): 66B02-mFc staining) in the peripheral blood of human CX3CR1-KI mice administered with the h1F8-2-2 antibody or BII00313.
Figure 13 shows the EAE scores obtained in a test in which the h1F8-2-2 antibody was administered to the human CX3CR1-KI mouse late-onset EAE model.
Figure 14 shows the arthritis scores obtained in a test in which the m1F8-2 antibody was administered to a human CX3CR1-KI mouse CAIA (anti-collagen antibody cocktail-induced arthritis) model.

### Description of Embodiments

The present invention will be described in more detail below. The scope of the present invention is not limited by the following descriptions, and any embodiments other than those illustrated below may also be carried out with appropriate modifications without departing from the spirit of the invention.

It should be noted that this specification incorporates the specification of Japanese Patent Application No. 2022-205800 (filed on December 22, 2022) in its entirety, based on which the present application claims priority. Moreover, all publications cited herein, including prior art documents, patent gazettes and other patent documents, are incorporated herein by reference.

### 1. Antibody against human CX3CR1 (anti-human CX3CR1 antibody)

### (1) Antigen preparation

Information about the amino acid sequence (SEQ ID NO: 17) of human CX3CR1 (hereinafter also referred to as hCX3CR1) has been registered and made public, e.g., on the website of Uniprot (https://www.uniprot.org/) under "Primary (citable) accession number: P49238-1" or on the website of NCBI (GenBank) (http://www.ncbi.nlm.nih.gov/) under "Accession number: NP_001328.1." It should be noted that information about the nucleotide sequence (SEQ ID NO: 16) encoding the amino acid sequence of hCX3CR1 has been registered and made public on the website of NCBI (GenBank) under "Accession number: NM_001337.3" (it should be noted that a sequence consisting of nucleotides 93 to 1160 in this nucleotide sequence registered and made public corresponds to the nucleotide sequence of SEQ ID NO: 16).

As an antigen, it is possible to use a polypeptide or peptide (hereinafter also simply referred to as a peptide) comprising the whole or a part of the amino acid sequence of hCX3CR1.

The peptide for use as an antigen may be prepared either by chemical synthesis or by synthesis through genetic engineering procedures using *E. coli* or the like, and techniques well known to those skilled in the art may be used for this purpose.

For chemical synthesis, the peptide may be synthesized by well-known peptide synthesis techniques. Moreover, the synthesis may be accomplished by applying either solid phase synthesis or liquid phase synthesis. A commercially available peptide synthesizer (e.g., CEM Japan: an automated parallel peptide synthesizer, MultiPep 2) may also be used for this purpose.

For peptide synthesis through genetic engineering procedures, DNA encoding the peptide is first designed and synthesized. The design and synthesis may be accomplished, for example, by PCR techniques using a vector or the like containing the full-length hCX3CR1 gene as a template and using primers which have been designed to allow synthesis of a desired DNA region. Moreover, based on the amino acid sequence of the peptide, gene synthesis may also be conducted to obtain DNA comprising a Kozak translation initiation sequence inserted at the 5'-terminal end and a translation termination codon inserted on the 3'-terminal side (e.g., using the service of GENEWIZ). Then, the above DNA may be ligated to an appropriate vector to obtain a recombinant vector for protein expression, and this recombinant vector may be introduced into a host, such that a desired gene can be expressed therein, thereby obtaining a transformant (Molecular cloning 4th Ed. Cold Spring Harbor Laboratory Press (2012)).

As a vector, a phage or plasmid which is autonomously replicable in host microorganisms is used. Further, it is also possible to use an animal virus or insect virus vector. To prepare a recombinant vector, purified DNA may be cleaved with an appropriate restriction enzyme and ligated to a vector by being inserted into, e.g., an appropriate restriction enzyme site in the vector DNA. There is no particular limitation on the host for use in transformation as long as it is capable of expressing a desired gene. Examples include bacteria (e.g., *E. coli, Bacillus subtilis),* yeast, animal cells (e.g., COS cells, CHO cells), insect cells or insects. It is also possible to use a mammal (e.g., goat) as a host. Procedures for introduction of a recombinant vector into a host are known. Moreover, the above transformant may be cultured, and the peptide for use as an antigen may be collected from the cultured product. The term "cultured product" is intended to mean either (a) a culture supernatant, or (b) cultured cells or cultured microorganisms, or a homogenate thereof.

After culture, when the desired peptide is produced within microorganisms or cells, the microorganisms or cells may be homogenized to thereby extract the peptide. Alternatively, when the desired peptide is produced outside microorganisms or cells, the cultured solution may be used directly or treated by centrifugation or other techniques to remove the microorganisms or cells. Then, the desired peptide may be isolated and purified by biochemical techniques commonly used for isolation and purification of peptides, as exemplified by ammonium sulfate precipitation, gel filtration, ion exchange chromatography, affinity chromatography and so on, which may be used either alone or in combination as appropriate.

The peptide for use as an antigen may also be obtained by *in vitro* translation using a cell-free synthesis system. In this case, it is possible to use two methods, i.e., a method in which RNA is used as a template and a method in which DNA is used as a template (transcription/translation). As a cell-free synthesis system, a commercially available system may be used, as exemplified by Expressway^{™} system (Invitrogen), PURESYSTEM^{®} (Post Genome Institute Co., Ltd., Japan), TNT system^{®} (Promega), etc.

The peptide obtained as described above may also be linked to an appropriate carrier protein such as bovine serum albumin (BSA), keyhole limpet hemocyanin (KLH), human thyroglobulin, avian gamma globulin, etc.

Moreover, the antigen may be a peptide consisting of an amino acid sequence with deletion, substitution or addition of one or more amino acids in the amino acid sequence of hCX3CR1 (SEQ ID NO: 17) or its partial sequence. For example, it is also possible to use a peptide consisting of an amino acid sequence with deletion of one or more (preferably one or several (e.g., 1 to 10, more preferably 1 to 5)) amino acids, with substitution of other amino acids for one or more (preferably one or several (e.g., 1 to 10, more preferably 1 to 5)) amino acids, or with addition of one or more (preferably one or several (e.g., 1 to 10, more preferably 1 to 5)) other amino acids in the amino acid sequence of hCX3CR1 or its partial sequence.

The gene to be introduced into cells or the like may be a gene encoding a hCX3CR1 protein or a partial fragment thereof or a mutated protein or fragment thereof. For example, it is possible to use a gene having the nucleotide sequence shown in SEQ ID NO: 16 or a partial sequence thereof for this purpose.

Alternatively, as a gene to be introduced into cells or the like, it is also possible to use a nucleotide sequence encoding a protein having the same activity or function as hCX3CR1, or a partial sequence thereof, which is hybridizable under stringent conditions with a sequence complementary to the nucleotide sequence shown in SEQ ID NO: 16.

The term "stringent conditions" refers to washing conditions after hybridization, and is intended to mean conditions where the salt (sodium) concentration in buffer is 10 to 500 mM and the temperature is 42°C to 72°C, preferably where the above salt concentration is 50 to 300 mM and the temperature is 55°C to 68°C.

For introduction of mutations into a gene, known techniques (e.g., Kunkel method or Gapped duplex method) may be used for this purpose. For example, it is possible to use a kit for mutation introduction based on site-directed mutagenesis, as exemplified by GeneArt^{™} Site-Directed Mutagenesis System (Invitrogen), TaKaRa Site-Directed Mutagenesis System (e.g., Mutan-K, Mutan-Super Express Km; Takara Bio Inc., Japan).

### (2) Preparation of polyclonal antibodies

The antigen prepared above is administered to a mammal for the purpose of immunization. Such a mammal is not limited in any way, and examples include rats, mice and rabbits, with mice being particularly preferred.

The amount of the antigen to be administered per animal may be determined, as appropriate, depending on the presence or absence of an adjuvant. Examples of an adjuvant include Freund's complete adjuvant (FCA), Freund's incomplete adjuvant (FIA), aluminum hydroxide adjuvant and so on. Immunization may be primarily accomplished by injection via the intravenous, footpad, subcutaneous or intraperitoneal route, etc. Moreover, the interval between immunizations is not limited in any way, and immunization may be repeated once to 10 times, preferably twice to three times, at intervals of several days to several weeks, preferably at intervals of 1 week. Further, at 3 to 7 days after the day of the final immunization, the animals are measured for their antibody titers by enzyme immunoassay (ELISA or EIA) or radioactive immunoassay (RIA), etc., and blood may be collected at the day when each animal shows the desired antibody titer, thereby obtaining antisera. In cases where antibodies are required to be purified in the above antibody collection procedures, known techniques such as salting out with ammonium sulfate, ion exchange chromatography, gel filtration chromatography, affinity chromatography and so on may be selected as appropriate or used in combination for purification purposes. Then, polyclonal antibodies in the antisera are measured for their reactivity by ELISA assay, etc.

### (3) Preparation of monoclonal antibody

### (3-1) Collection of antibody-producing cells

The anti-hCX3CR1 antibody of the present invention is not limited in any way, but is preferably a monoclonal antibody.

The antigen prepared above is administered to a mammal (e.g., rat, mouse, rabbit) for the purpose of immunization. The amount of the antigen to be administered per animal may be determined, as appropriate, depending on the presence or absence of an adjuvant. Examples of an adjuvant are the same as described above. Immunization procedures are also the same as described above. Further, at 1 to 60 days, preferably 1 to 14 days, after the day of the final immunization, antibody-producing cells are collected. Antibody-producing cells may be exemplified by spleen cells, lymph node cells and peripheral blood cells, etc., with lymph node cells or spleen cells being particularly preferred.

### (3-2) Cell fusion

To obtain hybridomas (antibody-producing cell lines), cell fusion is conducted between antibody-producing cells and myeloma cells. As myeloma cells to be fused with antibody-producing cells, it is possible to use generally available established cell lines of mouse or other animal origin. Cell lines preferred for use are those having drug selectivity and having the property of not surviving in HAT selective medium (i.e., a medium containing hypoxanthine, aminopterin and thymidine) in an unfused state, but surviving only when fused with antibody-producing cells.

Examples of myeloma cells include mouse myeloma cell lines, as exemplified by P3-X63-Ag8.653, P3-X63-Ag8(X63), P3-X63-Ag8.U1(P3U1), P3/NS I/1-Ag4-1(NS1) and Sp2/0-Ag14(Sp2/0), etc. Myeloma cells may be selected as appropriate in consideration of their compatibility with antibody-producing cells.

Then, myeloma cells and antibody-producing cells are provided for cell fusion. For cell fusion, in a serum-free medium for animal cell culture (e.g., DMEM or RPMI-1640 medium), 1 × 10⁶ to 1 × 10⁷/mL of antibody-producing cells are mixed with 2 × 10⁵ to 2 × 10⁶/mL of myeloma cells. The ratio of antibody-producing cells to myeloma cells (antibody-producing cells:myeloma cells) is not limited in any way, but is usually set to preferably 1:1 to 10:1, more preferably 3:1. Then, fusion reaction is conducted in the presence of a cell fusion promoter. As a cell fusion promoter, it is possible to use polyethylene glycol having an average molecular weight of 1,000 to 6,000 daltons (D), etc. Alternatively, a commercially available cell fusion apparatus using electrical stimulation (e.g., electroporation) may also be used to cause cell fusion between antibody-producing cells and myeloma cells.

### (3-3) Screening and cloning of hybridomas

After cell fusion, the cells are screened to select desired hybridomas. For screening, a cell suspension may be diluted as appropriate with, e.g., RPMI-1640 medium containing fetal bovine serum and then seeded on microtiter plates, and a selective medium may be added to each well, followed by culture while replacing the selective medium as appropriate. As a result, cells growing at around 14 days after the initiation of culture in the selective medium may be obtained as hybridomas.

Subsequently, the growing hybridomas are screened as to whether an antibody reactive to hCX3CR1 is present in their culture supernatants. Screening of these hybridomas is not limited in any way and may be conducted in accordance with commonly used procedures. For example, aliquots of the culture supernatants contained in the wells where hybridomas have grown may be sampled and screened by ELISA, EIA and RIA, etc.

The fused cells may be cloned by limiting dilution or other techniques. An antibody strongly reactive to hCX3CR1 is determined by flow cytometry or other techniques, and a hybridoma producing this antibody is selected and established as a clone.

### (3-4) Collection of monoclonal antibody

For culture of the establish hybridoma and collection of a monoclonal antibody from the resulting cultured product, commonly used procedures, e.g., cell culture-based procedures or ascites formation procedures may be used for this purpose. The term "culture" is intended to mean that a hybridoma is allowed to grow in a culture dish or a culture bottle, or that a hybridoma is allowed to proliferate in the abdominal cavity of an animal as described below.

In cell culture-based procedures, the hybridoma may be cultured in an animal cell culture medium (e.g., 10% fetal bovine serum-containing RPMI-1640 medium, MEM medium or serum-free medium) under standard culture conditions (e.g., 37°C, 5% CO₂ concentration) for 7 to 14 days to obtain an antibody from its culture supernatant.

In the case of ascites formation procedures, the hybridoma may be intraperitoneally administered at about 1 × 10⁷ cells to an animal of the same species as the mammal from which myeloma cells are derived, whereby the hybridoma is allowed to proliferate in abundance. Then, its ascites is preferably collected after 2 to 3 weeks.

In cases where the antibody is required to be purified in the above antibody collection procedures, known techniques such as salting out with ammonium sulfate, ion exchange chromatography, gel filtration, affinity chromatography and so on may be selected as appropriate or used in combination for purification purposes.

### (3-5) Activity and function of clones

(3-5-1) The anti-hCX3CR1 antibody of the present invention is preferably an antibody having inhibitory or suppressive activity against the interaction (preferably binding) between human CX3CR1 and human CX3CL1 (fractalkine), by way of example.

The inhibitory or suppressive activity (%) against the interaction between hCX3CR1 and fractalkine can be calculated, for example, according to the following equation. Inhibitory or suppressive activity (%) = 100 - (background-corrected reactivity in the presence of antibody / background-corrected reactivity in the absence of antibody) × 100
(3-5-2) The anti-hCX3CR1 antibody of the present invention is preferably an antibody having inhibitory or suppressive activity against the function of Eomes-expressing Th cells (helper T cells), by way of example. Moreover, the anti-hCX3CR1 antibody of the present invention is also preferably an antibody having inhibitory or suppressive activity against the function of Granzyme B-expressing Th cells, or an antibody having inhibitory or suppressive activity against the function of Eomes- and Granzyme B-expressing Th cells, or an antibody having inhibitory or suppressive activity against the function of Eomes- and/or IFN-γ-expressing Th cells, by way of example. The function intended herein (i.e., the function of Eomes- and/or Granzyme B-expressing Th cells, or the function of Eomes- and/or IFN-γ-expressing Th cells) is exemplified by infiltration into the CNS (central nervous system), etc.

The inhibitory or suppressive activity (%) against the function of Eomes- and/or Granzyme B-expressing Th cells or Eomes- and/or IFN-γ-expressing Th cells can be calculated, for example, according to the following equation. Inhibitory or suppressive activity (%) = 100 - (background-corrected reactivity in the presence of antibody / background-corrected reactivity in the absence of antibody) × 100
(3-5-3) The anti-hCX3CR1 antibody of the present invention is preferably an antibody having the function of improving experimental autoimmune encephalomyelitis (EAE) pathology or suppressing the progression of EAE pathology, by way of example.

EAE pathology refers to a condition caused in C57BL/6 mice administered with an MOG peptide and complete Freund's adjuvant (CFA) containing killed *Mycobacterium tuberculosis* H37Ra, while an EAE model is a model (test system) in which MS pathology in humans is reproduced in mice and evaluated for its severity in accordance with criteria in numerical form.

Symptoms and evaluation criteria for EAE are as defined below.

### <Evaluation criteria for EAE>

0: no clinical sign
1: partial tail paralysis
2: flaccid tail
3: partial hind limb paralysis
4: complete hind limb paralysis
5: hind limb and forelimb paralysis

The efficacy of the anti-hCX3CR1 antibody of the present invention having the function of improving MS or suppressing the progression of MS may be evaluated in this EAE model or in a late-onset EAE model as described later.

The late-onset EAE model used in the present invention is a special mouse model in which only the symptoms of progressive MS are experimentally reproduced, and is constructed using NR4A2-deficient mice. Further, in the present invention, to confirm the reactivity of 1F8 binding to hCX3CR1 in this model, NR4A2-deficient mice crossed with hCX3CR1-knocked-in mice are used (a human CX3CR1-KI mouse late-onset EAE model).

In more detail, the late-onset EAE model does not show an increase in EAE score, which is seen in the early stage after EAE induction in an EAE test using normal mice not deficient in NR4A2. On the other hand, after a certain period of time has passed after EAE induction, only an increase in EAE score arising from progressive MS is observed (i.e., EAE pathology). For this reason, the late-onset EAE model may be used particularly for evaluating progressive MS.
(3-5-4) The anti-hCX3CR1 antibody of the present invention is preferably an antibody which may be used in the prevention, suppression of progression, or treatment of progressive immune-mediated demyelinating diseases, by way of example.

The progressive immune-mediated demyelinating diseases intended here are preferably exemplified by progressive immune-mediated demyelinating diseases of the central nervous system. Such progressive immune-mediated demyelinating diseases may be exemplified by progressive multiple sclerosis (MS), and progressive MS is known to include primary progressive MS (PPMS), secondary progressive MS (SPMS) in which RRMS pathology continues for a certain period of time and then progresses into advanced pathology, and progressive relapsing MS (PRMS) in which the pathology progresses with repeated relapses (see Patent Document 3). However, the anti-hCX3CR1 antibody of the present invention is not limited to these diseases, and may be used in the prevention, suppression of progression, or treatment of any one or more of, for example, autoimmune diseases (e.g., rheumatoid arthritis, inflammatory bowel disease, Crohn's disease, systemic scleroderma, systemic lupus erythematosus, and autoimmune myositis), heart diseases including atherosclerosis, chronic renal diseases, diabetic heart diseases, diabetic renal diseases, interstitial lung diseases, cancers (e.g., breast cancer, prostate cancer, colorectal cancer), COVID-19 (novel coronavirus infectious disease)-related central nervous system diseases, as well as neurodegenerative diseases (e.g., ALS, Alzheimer's disease, and multiple system atrophy).
(3-5-5) The anti-hCX3CR1 antibody of the present invention preferably has a dissociation constant (Kd value) of 1.0 × 10⁻¹⁰ M or less, more preferably 1.0 × 10⁻¹¹ M or less, and even more preferably 1.0 × 10⁻¹² M or less, without being limited thereto.

The antibody's binding ability (affinity) may be measured as a dissociation constant (Kd value), dissociation rate constant (Kdiss [1/Sec]) or association rate constant (Kass [1/M.Sec]), for example, by Scatchard analysis or with a surface plasmon resonance sensor called Biacore. Examples of a Biacore apparatus include Biacore 3000, Biacore 2000, Biacore X, Biacore J, Biacore Q (all from Biacore) and so on. The antibody is preferred in terms of having higher binding ability (affinity) at a lower dissociation constant (Kd value). The Kd value is determined by two parameters, i.e., Kdiss and Kass, and can be expressed by the equation: Kd [M] = Kdiss/Kass, by way of example.

### (3-6) Epitope for anti-hCX3CR1 antibody

An epitope (antigenic determinant) for the anti-hCX3CR1 antibody in the present invention may be a region of at least a part of the antigen hCX3CR1. For example, preferred is a region of at least a part of the extracellular domain of hCX3CR1, but any region other than the extracellular domain may also be an epitope.

It should be noted that antibodies capable of binding to an epitope region to which the anti-hCX3CR1 antibody in the present invention binds (recognizes) also fall within the present invention.

### (4) Genetically recombinant antibodies and antibody fragments

### (4-1) Genetically recombinant antibodies

A preferred embodiment of the anti-hCX3CR1 antibody of the present invention may be a genetically recombinant antibody. Examples of a genetically recombinant antibody include, but are not limited to, a chimeric antibody, a humanized antibody, and a human antibody (a complete human antibody), etc.

A chimeric antibody (i.e., a humanized chimeric antibody) is an antibody in which antibody variable regions of mouse origin are linked (conjugated) to constant regions of human origin (see, e.g., Proc. Natl. Acad. Sci. U.S.A. 81, 6851-6855 (1984)). For preparation of a chimeric antibody, gene recombination technology may be used for easy construction such that the thus linked antibody is obtained.

For preparation of a humanized antibody, for example, complementarity determining regions (CDRs) from mouse antibody variable regions are grafted into human variable regions to prepare reconstituted variable regions whose framework regions (FRs) are of human origin and whose CDRs are of mouse origin (so-called CDR grafting). Then, these humanized reconstituted human variable regions are linked to human constant regions. Procedures for preparation of such a humanized antibody have been well known in the art (see Nature, 321, 522-525 (1986); J. Mol. Biol., 196, 901-917 (1987); Queen C et al., Proc. Natl. Acad. Sci. USA, 86: 10029-10033 (1989); JP H04-502408 A (Japanese Patent No. 2828340; Queen et al.), etc.).

A human antibody (complete human antibody) is usually an antibody in which hyper variable regions serving as antigen-binding sites in the V regions, the other portions in the V regions and the constant regions have the same structures as those of an antibody of human origin. However, hyper variable regions may be of other animal origin. Techniques to prepare a human antibody have also been known, and a method through genetic engineering procedures has been established for the preparation of gene sequences common to humans. Such a human antibody may be obtained, for example, by using human antibody-producing mice carrying human chromosome fragments containing genes for human antibody H and L chains (see, e.g., Tomizuka, K. et al., Nature Genetics, (1977) 16, 133-143; Kuroiwa, Y. et al., Nuc. Acids Res., (1998) 26, 3447-3448; Yoshida, H. et al., Animal Cell Technology: Basic and Applied Aspects, (1999) 10, 69-73 (Kitagawa, Y., Matuda, T. and Iijima, S. eds.), Kluwer Academic Publishers; Tomizuka, K. et al., Proc. Natl. Acad. Sci. USA, (2000) 97, 722-727) or by screening human antibody libraries to obtain a phage display-derived human antibody (see, e.g., Wormstone, I. M. et al, Investigative Ophthalmology & Visual Science., (2002) 43 (7), 2301-8; Carmen, S. et al., Briefings in Functional Genomics and Proteomics, (2002) 1 (2), 189-203; Siriwardena, D. et al., Opthalmology, (2002) 109 (3), 427-431).

Alternatively, as a human antibody, it is also possible to use an antibody produced (and further purified) from a clone specifically binding to a desired antigen (i.e., hCX3CR1 in the present invention) by using the human ADLib technique (the library shown in WO 2015/167011), or a variant thereof with modifications (preferably amino acid substitutions) in the amino acid sequence of this antibody (particularly the amino acid sequence(s) of VH and/or VL, preferably at least one CDR sequence in VH and/or VL).

The above chimeric, humanized and human antibodies are not limited in any way, but are configured such that the N-glycoside-linked complex sugar chain in the antibody Fc region preferably has no fucose linked to N-acetylglucosamine at the reducing terminal of the sugar chain, as specifically exemplified by antibodies composed of genetically recombinant antibody molecules whose Fc region has a sugar chain in which the 1-position of the fucose is not α-liked to the 6-position of N-acetylglucosamine at the reducing terminal of the N-glycoside-linked complex sugar chain. Such an antibody allows an improvement in ADCC activity. It should be noted that this point (i.e., the characteristics of the N-glycoside-linked complex sugar chain in the antibody Fc region) is also preferred for the above polyclonal and monoclonal antibodies.

Preferred examples of the anti-hCX3CR1 antibody of the present invention include those in which
the amino acid sequences of heavy chain variable region (VH) complementarity determining region (CDR) 1 (CDR-H1), CDR2 (CDR-H2) and CDR3 (CDR-H3) consist of:
the amino acid sequences shown in SEQ ID NOs: 2, 3 and 4, respectively, and
the amino acid sequences of light chain variable region (VL) CDR1 (CDR-L1), CDR2 (CDR-L2) and CDR3 (CDR-L3) consist of:
   the amino acid sequences shown in SEQ ID NOs: 6, 7 and 8, respectively; or
   the amino acid sequences shown in SEQ ID NOs: 10, 7 and 8, respectively.

In more detail, preferred examples of the anti-hCX3CR1 antibody of the present invention include those in which
(a)
   the amino acid sequences of CDR-H1, CDR-H2 and CDR-H3 consist of the amino acid sequences shown in SEQ ID NOs: 2, 3 and 4, respectively, and
   the amino acid sequences of CDR-L1, CDR-L2 and CDR-L3 consist of the amino acid sequences shown in SEQ ID NOs: 6, 7 and 8, respectively, as well as
(b)
   the amino acid sequences of CDR-H1, CDR-H2 and CDR-H3 consist of the amino acid sequences shown in SEQ ID NOs: 2, 3 and 4, respectively, and
   the amino acid sequences of CDR-L1, CDR-L2 and CDR-L3 consist of the amino acid sequences shown in SEQ ID NOs: 10, 7 and 8, respectively.

Moreover, more preferred embodiments of the anti-hCX3CR1 antibody of the present invention include those in which
the amino acid sequence of the heavy chain variable region (VH) consists of the amino acid sequence shown in SEQ ID NO: 1, 11, 12, 13 or 14, and
the amino acid sequence of the light chain variable region (VL) consists of the amino acid sequence shown in SEQ ID NO: 5, 9 or 15.

In more detail, preferred examples of the anti-hCX3CR1 antibody of the present invention include those in which
(a)
   the amino acid sequence of VH consists of the amino acid sequence shown in SEQ ID NO: 1, and
   the amino acid sequence of VL consists of the amino acid sequence shown in SEQ ID NO: 5,
(b)
   the amino acid sequence of VH consists of the amino acid sequence shown in SEQ ID NO: 1, and
   the amino acid sequence of VL consists of the amino acid sequence shown in SEQ ID NO: 9,
(c)
   the amino acid sequence of VH consists of the amino acid sequence shown in SEQ ID NO: 11, and
   the amino acid sequence of VL consists of the amino acid sequence shown in SEQ ID NO: 15,
(d)
   the amino acid sequence of VH consists of the amino acid sequence shown in SEQ ID NO: 12, and
   the amino acid sequence of VL consists of the amino acid sequence shown in SEQ ID NO: 15,
(e)
   the amino acid sequence of VH consists of the amino acid sequence shown in SEQ ID NO: 13, and
   the amino acid sequence of VL consists of the amino acid sequence shown in SEQ ID NO: 15, as well as
(f)
   the amino acid sequence of VH consists of the amino acid sequence shown in SEQ ID NO: 14, and
   the amino acid sequence of VL consists of the amino acid sequence shown in SEQ ID NO: 15.

It should be noted that among the antibodies shown in the Example section described later as embodiments of the anti-hCX3CR1 antibody of the present invention, m1F8-1, m1F8-2, h1F8-2-1, h1F8-2-2, h1F8-2-3 and h1F8-2-4 antibodies are summarized in Table A below as to the amino acid sequences of their VH and VL, and CDR-H1 to CDR-H3 and CDR-L1 to CDR-L3, which consist of the amino acid sequences of SEQ ID NOs indicated in Table A below.

**[Table A]**

| Antibody name | VH | CDR-H1 | CDR-H2 | CDR-H3 | VL | CDR-L1 | CDR-L2 | CDR-L3 |
|---|---|---|---|---|---|---|---|---|
| m1F8-1 | SEQ ID NO: 1 | SEQ ID NO: 2 | SEQ ID NO: 3 | SEQ ID NO: 4 | SEQ ID NO: 5 | SEQ ID NO: 6 | SEQ ID NO: 7 | SEQ ID NO: 8 |
| m1F8-2 | SEQ ID NO: 1 | SEQ ID NO: 2 | SEQ ID NO: 3 | SEQ ID NO: 4 | SEQ ID NO: 9 | SEQ ID NO: 10 | SEQ ID NO: 7 | SEQ ID NO: 8 |
| h1F8-2-1 | SEQ ID NO: 11 | SEQ ID NO: 2 | SEQ ID NO: 3 | SEQ ID NO: 4 | SEQ ID NO: 15 | SEQ ID NO: 10 | SEQ ID NO: 7 | SEQ ID NO: 8 |
| h1F8-2-2 | SEQ ID NO: 12 | SEQ ID NO: 2 | SEQ ID NO: 3 | SEQ ID NO: 4 | SEQ ID NO: 15 | SEQ ID NO: 10 | SEQ ID NO: 7 | SEQ ID NO: 8 |
| h1F8-2-3 | SEQ ID NO: 13 | SEQ ID NO: 2 | SEQ ID NO: 3 | SEQ ID NO: 4 | SEQ ID NO: 15 | SEQ ID NO: 10 | SEQ ID NO: 7 | SEQ ID NO: 8 |
| h1F8-2-4 | SEQ ID NO: 14 | SEQ ID NO: 2 | SEQ ID NO: 3 | SEQ ID NO: 4 | SEQ ID NO: 15 | SEQ ID NO: 10 | SEQ ID NO: 7 | SEQ ID NO: 8 |

### (4-2) Antibody fragments

A fragment (partial fragment) of the anti-hCX3CR1 antibody of the present invention also falls within the antibody of the present invention. The antibody fragment of the present invention has binding activity to hCX3CR1 (i.e., is capable of binding to hCX3CR1), as in the case of the anti-hCX3CR1 antibody of the present invention. Preferably, the antibody fragment of the present invention has inhibitory or suppressive activity against the interaction or binding between human CX3CR1 and human CX3CL1 (fractalkine), has inhibitory or suppressive activity against the function (e.g., infiltration into the CNS) of Eomes- and/or Granzyme B-expressing Th cells or Eomes- and/or IFN-γ-expressing Th cells, has the function of improving EAE pathology or suppressing the progression of EAE pathology, or can be used in the prevention, suppression of progression, or treatment of progressive immune-mediated demyelinating diseases (preferably progressive immune-mediated demyelinating diseases of the central nervous system, e.g., secondary progressive multiple sclerosis (SPMS)), as described above.

A fragment of the antibody is intended to mean a partial region of the anti-hCX3CR1 polyclonal antibody or the anti-hCX3CR1 monoclonal antibody (i.e., an antibody fragment derived from the anti-hCX3CR1 antibody of the present invention), and examples include Fab, Fab', F(ab')₂, Fv (variable fragment of antibody), single chain antibody (e.g., H chain, L chain, H chain V region, and L chain V region), scFv, diabody (scFv dimer), dsFv (disulfide stabilized V region), as well as peptides at least partially containing complementarity determining regions (CDRs), etc.

Fab is an antibody fragment having a molecular weight of about 50,000 and having antigen binding activity, which is composed of the N-terminal half of H chain and the full length of L chain linked via a disulfide bond, among fragments obtained by treating an antibody molecule with a protease, papain. Alternatively, Fab may also be prepared as follows: DNA encoding antibody Fab is inserted into a prokaryotic or eukaryotic expression vector, and this vector is introduced into a prokaryotic or eukaryotic organism for expression.

F(ab')₂ is an antibody fragment having a molecular weight of about 100,000 and having antigen binding activity, which is slightly larger than that composed of Fab fragments linked via disulfide bonds in the hinge region, among fragments obtained by treating an antibody molecule with a protease, pepsin. Alternatively, F(ab')₂ may also be prepared by linking Fab' fragments described later via thioether bonds or disulfide bonds.

Fab' is an antibody fragment having a molecular weight of about 50,000 and having antigen binding activity, which is obtained by cleaving the disulfide bonds in the hinge region of the above F(ab')₂. Alternatively, Fab' may also be prepared as follows: DNA encoding an antibody Fab' fragment is inserted into a prokaryotic or eukaryotic expression vector, and this vector is introduced into a prokaryotic or eukaryotic organism for expression.

scFv is an antibody fragment having antigen binding activity, which is composed of a single heavy chain variable region (VH) and a single light chain variable region (VL) linked via an appropriate peptide linker (P), i.e., a VH-P-VL or VL-P-VH polypeptide. For preparation of scFv, cDNAs encoding antibody VH and VL may be obtained to construct DNA encoding scFv, and this DNA may be inserted into a prokaryotic or eukaryotic expression vector, followed by introducing this expression vector into a prokaryotic or eukaryotic organism for expression.

Diabody is an antibody fragment composed of dimerized scFv fragments and having divalent antigen binding activity. The divalent antigen binding activity may be directed to the same antigen or to different antigens. For preparation of diabody, cDNAs encoding antibody VH and VL may be obtained to construct DNA encoding scFv such that the amino acid sequence of a peptide linker (P) has a length of 8 residues or less, and this DNA may be inserted into a prokaryotic or eukaryotic expression vector, followed by introducing this expression vector into a prokaryotic or eukaryotic organism for expression.

dsFv is an antibody fragment composed of VH and VL polypeptides, in each of which a single amino acid residue is replaced with a cysteine residue and which are linked via a disulfide bond between these cysteine residues. An amino acid residue to be replaced with a cysteine residue can be selected based on three-dimensional structure prediction of antibody according to the method reported by Reiter et al. (Protein Engineering, 7, 697-704, 1994). For preparation of dsFv, cDNAs encoding antibody VH and VL may be obtained to construct DNA encoding dsFv, and this DNA may be inserted into a prokaryotic or eukaryotic expression vector, followed by introducing this expression vector into a prokaryotic or eukaryotic organism for expression.

A CDR-containing peptide is configured to comprise at least one or more regions of VH CDRs (CDR-H1 to CDR-H3) and VL CDRs (CDR-L1 to CDR-L3), more preferably configured to comprise all VH CDRs or all VL CDRs, and particularly preferably configured to comprise all VH and VL CDRs (i.e., 6 regions in total). The amino acid sequences of CDRs are preferably exemplified by the various amino acid sequences of CDR-H1 to CDR-H3 and CDR-L1 to CDR-L3 mentioned above. In the case of a peptide containing a plurality of CDRs, these CDRs may be linked directly or through an appropriate peptide linker. For preparation of a CDR-containing peptide, DNA encoding CDR in antibody VH or VL may be constructed, and the DNA may be inserted into a prokaryotic or eukaryotic expression vector, followed by introducing this expression vector into a prokaryotic or eukaryotic organism for expression. Alternatively, a CDR-containing peptide may also be prepared by chemical synthesis such as Fmoc (fluorenylmethyloxycarbonyl) and tBoc (t-butyloxycarbonyl) methods.

The antibody fragments of the present invention may be antibody fragments comprising a part or the whole of the antibody Fc region whose N-glycoside-linked complex sugar chain has no fucose linked to N-acetylglucosamine at the reducing terminal of the sugar chain, or alternatively, may be fusion proteins of the antibody fragments mentioned above with a part or the whole of the antibody Fc region whose N-glycoside-linked complex sugar chain has no fucose linked to N-acetylglucosamine at the reducing terminal of the sugar chain. Such antibody fragments allow a dramatic improvement in ADCC activity and are therefore preferred.

In the following descriptions in this specification, the above antibody fragments also fall within the anti-hCX3CR1 antibody of the present invention.

### 2. Polynucleotide, recombinant vector and transformant

The present invention also enables the provision of a polynucleotide (gene, DNA) encoding the above anti-hCX3CR1 antibody of the present invention or an antibody fragment thereof. In more detail, such a polynucleotide is preferably a polynucleotide comprising a nucleotide sequence encoding any of the amino acid sequences shown as examples for the above anti-hCX3CR1 antibody of the present invention or an antibody fragment thereof. Moreover, the polynucleotide of the present invention may consist only of a polynucleotide encoding the anti-hCX3CR1 antibody of the present invention or an antibody fragment thereof, or may comprise, in addition to this polynucleotide, known nucleotide sequences (e.g., a transcription promoter, an SD sequence, a Kozak sequence, a terminator) required for gene expression, without being limited thereto.

Such a polynucleotide encoding the anti-hCX3CR1 antibody of the present invention or an antibody fragment thereof has no particular limitation on its codons corresponding to individual amino acids after translation, and may comprise nucleotide DNA showing codons commonly used (preferably codons frequently used) in humans or other mammals after transcription or may comprise nucleotide DNA showing codons commonly used (preferably codons frequently used) in microorganisms (e.g., *E. coli* or yeast) or plants, etc., after transcription.

The present invention also enables the provision of a recombinant vector comprising the above polynucleotide of the present invention and a transformant comprising the recombinant vector.

A polynucleotide (gene, DNA) to be integrated into an expression vector used as a recombinant vector may optionally be pre-ligated upstream with a transcription promoter, an SD sequence (when prokaryotic cells are used as a host) and a Kozak sequence (when eukaryotic cells are used as a host) and pre-ligated downstream with a terminator, and may also be pre-ligated with an enhancer, a splicing signal, a poly-A addition signal, a selective marker, etc. It should be noted that individual elements required for gene expression including a transcription promoter as mentioned above may be contained initially in the polynucleotide, or alternatively, those contained originally in an expression vector, if any, may be used. There is no particular limitation on the mode of use of each element.

For integration of such a polynucleotide into an expression vector, various known techniques based on gene recombination technology may be used, including those using restriction enzymes, those using topoisomerases, etc. Moreover, an expression vector is not limited in any way as long as it may carry a polynucleotide (gene, DNA) encoding the anti-hCX3CR1 antibody of the present invention or an antibody fragment thereof, as exemplified by plasmid DNA, bacteriophage DNA, retrotransposon DNA, a retroviral vector, artificial chromosomal DNA, etc., and a vector suitable for host cells to be used may be selected as appropriate for use.

Then, the above recombinant vector thus constructed may be introduced into a host to obtain a transformant, and this transformant may be cultured to allow the expression of the anti-hCX3CR1 antibody of the present invention or an antibody fragment thereof. It should be noted that the term "transformant" in the context of the present invention is intended to mean a host into which a foreign gene has been introduced, and examples include a host into which a foreign gene has been introduced by introduction of plasmid DNA or the like (transformation) and a host into which a foreign gene has been introduced by infection with various viruses and phages (transduction).

Such a host is not limited in any way and may be selected as appropriate as long as it allows the expression of the anti-hCX3CR1 antibody of the present invention or an antibody fragment thereof after introduction of the above recombinant vector, and examples include known hosts such as various animal cells (e.g., human and mouse cells), various plant cells, bacteria, yeast, plant cells, etc.

When animal cells are used as a host, examples include human fibroblasts, human fetal kidney cells, HEK293 cells, 293F cells, CHO cells, monkey cells COS-7, Vero, mouse L cells, rat GH3, human FL cells, etc. Alternatively, insect cells such as Sf9 cells and Sf21 cells may also be used.

When bacteria are used as a host, examples include *E. coli, Bacillus subtilis,* etc.

When yeast is used as a host, examples include *Saccharomyces cerevisiae, Schizosaccharomyces pombe,* etc.

When plant cells are used as a host, examples include tobacco BY-2 cells, etc.

Procedures for obtaining a transformant are not limited in any way and may be selected as appropriate in consideration of the combination between host and expression vector types. Preferred examples include electroporation, lipofection, heat shock transfection, PEG transfection, calcium phosphate transfection, DEAE-dextran transfection, and infection with various viruses (e.g., DNA virus, RNA virus).

In the resulting transformant, the codon types of the polynucleotide contained in the recombinant vector are not limited in any way and may be either identical with or different from the codon types in the host actually used.

### 3. Pharmaceutical composition and others

The anti-hCX3CR1 antibody of the present invention (including a fragment derived therefrom) is useful as an active ingredient contained in a pharmaceutical composition.

For example, the anti-hCX3CR1 antibody of the present invention is preferred for use in the prevention, suppression of progression, and/or treatment of progressive immune-mediated demyelinating diseases. Thus, the pharmaceutical composition is useful as a pharmaceutical composition for the prevention, suppression of progression, and/or treatment of progressive immune-mediated demyelinating diseases. Namely, the anti-hCX3CR1 antibody of the present invention is useful as an active ingredient contained in a prophylactic agent, a progression suppressor and/or a therapeutic agent for progressive immune-mediated demyelinating diseases. As to progressive immune-mediated demyelinating diseases to be applied by the pharmaceutical composition of the present invention, the explanations about their specific examples or the like described above can also be applied.

Further, the pharmaceutical composition of the present invention may be a composition which is used to inhibit or suppress the interaction or binding between human CX3CR1 and human CX3CL1 (fractalkine), or may be a composition which is used to inhibit or suppress the function (e.g., infiltration into the CNS) of Eomes- and/or Granzyme B-expressing Th cells or Eomes- and/or IFN-γ-expressing Th cells, or may be a composition which is used to improve EAE pathology or suppress the progression of EAE pathology.

The pharmaceutical composition of the present invention is preferably provided in the form of a pharmaceutical composition comprising the anti-hCX3CR1 antibody of the present invention as an active ingredient and further comprising a pharmaceutically acceptable carrier. Moreover, the pharmaceutical composition of the present invention may also be combined with known drugs (e.g., known drugs for use in the prevention, suppression of progression, and/or treatment of progressive immune-mediated demyelinating diseases). The embodiment of combined use is not limited in any way, and may be, for example, an embodiment where the pharmaceutical composition of the present invention further comprises such a known drug, or an embodiment where the pharmaceutical composition of the present invention is used in combination with such a known drug. Such combined use gives a higher prophylactic, progression suppressive and/or therapeutic effect.

The term "pharmaceutically acceptable carrier" is intended to include an excipient, a diluent, an extender, a disintegrant, a stabilizer, a preservative, a buffering agent, an emulsifier, an aromatic, a coloring agent, a sweetener, a thickener, a corrective, a solubilizer or other additives, etc. When using one or more of such carriers, a pharmaceutical composition can be prepared in the form of an injection, a solution, a capsule, a suspension, an emulsion or a syrups, etc. These pharmaceutical compositions may be administered orally or parenterally. Other forms for parenteral administration include an injection comprising one or more active substances and formulated in a standard manner. In the case of an injection, it may be prepared by being dissolved or suspended in a pharmaceutically acceptable carrier such as physiological saline or commercially available distilled water for injection, etc.

When antibody fragments (particularly small antibody fragments) derived from the anti-hCX3CR1 antibody of the present invention are administered *in vivo, a* colloidal dispersion system may be used, in addition to the forgoing embodiments. A colloidal dispersion system can be expected to exert the effect of enhancing the *in vivo* stability of a compound (antibody fragment) and the effect of efficiently delivering a compound to a particular organ, tissue or cell. Such a colloidal dispersion system is not limited in any way as long as it is commonly used, and examples include polyethylene glycol, a polymer complex, a polymer aggregate, a nanocapsule, a microsphere, a bead, and lipid-based dispersion systems including an oil-in-water emulsion, a micelle, a mixed micelle and a liposome. Preferred are several types of liposomes and artificial membrane vesicles having the effect of efficiently delivering a compound to a particular organ, tissue or cell (Mannino et al., Biotechniques, 1988, 6, 682; Blume and Cevc, Biochem. et Biophys. Acta, 1990, 1029, 91; Lappalainen et al., Antiviral Res., 1994, 23, 119; Chonn and Cullis, Current Op. Biotech., 1995, 6, 698).

The dose of the pharmaceutical composition of the present invention will vary depending on the age, sex, body weight and symptom of a target patient, the therapeutic effect, the mode of administration, the time required for treatment, or the type of the anti-hCX3CR1 antibody of the present invention to be contained in the pharmaceutical composition, etc. In general, the pharmaceutical composition of the present invention may be administered at a single dose ranging from 600 µg to 6000 mg per adult, without being limited thereto.

For example, when administered as an injection, the pharmaceutical composition of the present invention may be administered to a human patient at a dose of 100 µg to 100 mg per kg body weight per dosing once to several times per day average, and preferably may be administered once every 3 days, 1 week, 10 days or 2 weeks or singly (i.e., the total number of administration is once). The mode of administration may be exemplified by intravenous injection, subcutaneous injection, intracutaneous injection, intramuscular injection or intraperitoneal injection, etc., but preferred is intravenous injection. Moreover, an injection may optionally be prepared as a non-aqueous dilution (e.g., polyethylene glycol, a vegetable oil sch as olive oil, an alcohol such as ethanol), a suspension or an emulsion. Such an injection may be sterilized by filtration sterilization through a filter, incorporation with a disinfectant, etc. These injections may be prepared in reconstitutable form. Namely, they may be converted into sterile solid compositions by freeze-drying or other techniques, and then dissolved in sterile distilled water for injection or other solvents before use.

It should be noted that the present invention provides the use of the anti-hCX3CR1 antibody of the present invention and/or the complex of the present invention for the manufacture of a medicament (drug) for treating, preventing and/or diagnosing tumors or inflammatory diseases and/or autoimmune diseases (hereinafter also referred to as tumors, etc.) or for inhibiting or suppressing the interaction (preferably binding) between hCX3CR1 and human CX3CL1.

Moreover, the present invention provides the anti-hCX3CR1 antibody of the present invention for the prevention, suppression of progression, and/or treatment of progressive immune-mediated demyelinating diseases, the anti-hCX3CR1 antibody of the present invention for inhibiting and/or suppressing the interaction or binding between human CX3CR1 and human CX3CL1 (fractalkine), the anti-hCX3CR1 antibody of the present invention for inhibiting and/or suppressing the function (e.g., infiltration into the CNS) of Eomes- and/or Granzyme B-expressing Th cells or Eomes- and/or IFN-γ-expressing Th cells, or the anti-hCX3CR1 antibody of the present invention for improving EAE pathology and/or suppressing the progression of EAE pathology.

The present invention further provides a method for the prevention, suppression of progression, and/or treatment of progressive immune-mediated demyelinating diseases, a method for inhibiting and/or suppressing the interaction or binding between human CX3CR1 and human CX3CL1 (fractalkine), a method for inhibiting and/or suppressing the function (e.g., infiltration into the CNS) of Eomes- and/or Granzyme B-expressing Th cells or Eomes- and/or IFN-γ-expressing Th cells, or a method for improving EAE pathology and/or suppressing the progression of EAE pathology, each method being characterized in that the anti-hCX3CR1 antibody of the present invention is used (i.e., administered to a subject (patient)). The present invention also provides the use of the anti-hCX3CR1 antibody of the present invention for the prevention, suppression of progression, and/or treatment of progressive immune-mediated demyelinating diseases, for inhibiting and/or suppressing the interaction or binding between human CX3CR1 and human CX3CL1 (fractalkine), for inhibiting and/or suppressing the function (e.g., infiltration into the CNS) of Eomes- and/or Granzyme B-expressing Th cells or Eomes- and/or IFN-γ-expressing Th cells, or for improving EAE pathology and/or suppressing the progression of EAE pathology.

### 4. Kit

The anti-hCX3CR1 antibody of the present invention can also be provided in the form of a kit for the prevention, suppression of progression, and/or treatment of progressive immune-mediated demyelinating diseases, a kit for inhibiting and/or suppressing the interaction or binding between human CX3CR1 and human CX3CL1 (fractalkine), a kit for inhibiting and/or suppressing the function (e.g., infiltration into the CNS) of Eomes- and/or Granzyme B-expressing Th cells or Eomes- and/or IFN-γ-expressing Th cells, or a kit for improving EAE pathology and/or suppressing the progression of EAE pathology. Moreover, the anti-hCX3CR1 antibody of the present invention can also be provided in the form of a diagnostic kit allowing the diagnosis of progressive immune-mediated demyelinating diseases. As to progressive immune-mediated demyelinating diseases, the explanations about their specific examples or the like described above can also be applied.

In addition to the anti-hCX3CR1 antibody of the present invention, the kit of the present invention may further comprise various buffers, sterilized water, various cell culture vessels, various reaction vessels (e.g., Eppendorf tubes), a blocking agent (bovine serum albumin (BSA), skimmed milk, goat serum or other serum components), a detergent, a surfactant, various plates, an antiseptic (e.g., sodium azide), and an instruction manual for experimental operations (manufacturer's instructions), etc.

### Examples

The present invention will be further described in more detail by way of the following examples, although the present invention is not limited only to these examples.

### [Example 1]

### 1. Establishment of monoclonal antibody against human CX3CR1 (hCX3CR1)

### 1-1. Preparation of hCX3CR1 expression vector and antigen

Based on the amino acid sequence of a hCX3CR1 protein (Uniprot registration number: P49238-1), a DNA sequence encoding this amino acid sequence was synthesized (using the service of Genscript) and inserted into a pCAGGS vector using an "In-Fusion HD Cloning Kit" (Takara Bio Inc., Japan). The DNA was amplified using a forward primer with a Kozak translation initiation sequence, a reverse primer with a translation termination codon and "Premix Ex Taq (Ex Taq version 2.0)" (Takara Bio Inc., Japan), and then inserted into a vector included in a "pEF6/V5-His TOPO TA Expression kit" (Takara Bio Inc., Japan) to obtain a hCX3CR1 expression vector (hCX3CR1_pEF6/V5-His). For preparation of an antigen for cell-mediated immunity and cells for activity evaluation, hCX3CR1_pEF6/V5-His was introduced into Ba/F3 cells through electroporation, and the cells were cultured in a blasticidin-containing selective medium and subjected to a flow cytometer to separate hCX3CR1-expressing cells, which were then used as a stable expression cell line (hCX3CR1-expressing Ba/F3 cells). Moreover, the same procedures were repeated based on the amino acid sequence of a mouse CX3CR1 (mCX3CR1) protein (Uniprot registration number: Q9Z0D9) and using "pUNO1-mCX3CR1" (Invitrogen) as a template to prepare a mCX3CR1 expression vector (mCX3CR1_pEF6/V5-His) and mCX3CR1-expressing Ba/F3 cells, which were then provided for cross-reactivity test and epitope analysis test.

For preparation of an antigen-expressing cell membrane fraction, a hCX3CR1 gene sequence derived from hCX3CR1_pEF6/V5-His was introduced into pcDNA3.3 (Thermo Fisher Scientific) to prepare an expression vector (hCX3CR1_pcDNA3.3). Then, hCX3CR1_pcDNA3.3 was used as a transfection plasmid to effect transient expression using an "Expi293 expression system" (Thermo Fisher Scientific). A membrane fraction was prepared from the cultured cells and suspended in PBS for use as a hCX3CR1-expressing cell membrane fraction (using the service of SEEDSUPPLY Inc., Japan), which was then used as an antigen.

Moreover, as a recombinant protein antigen, a fusion protein was produced using an Expi293 system such that the N-terminal extracellular domain sequence of the hCX3CR1 protein (DQFPESVTENFEYDDLAEACYIGDIVVFGT (i.e., a sequence at positions 2 to 31 in the amino acid sequence of SEQ ID NO: 17)) was fused at the N-terminal end with GST or with the Fc region of human or mouse antibody, followed by purification to obtain the purified protein.

### 1-2. Immunization of animals

For antibody preparation, various hCX3CR1 antigens thus prepared were immunized into ICR mice (Sankyo Labo Service Co., Ltd., Japan, CLEA Japan, Inc., Japan), etc., on the basis of the following procedure.

The antigens were each mixed with an adjuvant "TiterMax Gold" (TiterMax) and administered into footpads such that the antigen dose per mouse was 200 µg. On Day 3 after administration and further at approximately weekly intervals, the mice were boosted in the same manner as above without the adjuvant such that the antigen dose per mouse was 10 to 50 µg.

### 1-3. Preparation of hybridomas

Lymph node cells or spleen cells isolated from the immunized mice were used to prepare monoclonal antibodies against hCX3CR1. After completion of the immunization, subinguinal, iliac, popliteal and other lymph nodes were collected from the mice to prepare cell suspensions, each of which was then mixed with SP2/0-Ag14 myeloma cells, followed by electrical cell fusion with an electrical cell fusion apparatus "ECFG21" (Nepa Gene Co., Ltd., Japan).

The fused cells were suspended in "ClonaCell^{™}-HY Medium D" (STEM CELL) and seeded on plastic dishes. After seeding, colonies formed at around 10 days were isolated into 96-well plastic plates containing a hybridoma medium dispensed in advance, and their culture supernatants were used for *in vitro* screening of antibodies. It should be noted that for use as a hybridoma medium, RPMI 1640 (Thermo Fisher Scientific) was supplemented with 1/50 volumes of Nutridoma-CS (Merck & Co., Inc.) and 1/50 volumes of HAT Supplement (Thermo Fisher Scientific).

### 1-4. Antibody screening in an in vitro test system

The screening of the resulting hybridoma clones was accomplished by the following two methods.

### 1-4-1. Screening based on binding activity

The culture supernatants of the hybridomas were evaluated by Cell ELISA for their reactivity to plates in which hCX3CR1-expressing rat hematopoietic cells "HTS015C" or non-hCX3CR1-expressing cells "HTSCHEM-1" (both from Eurofins) had been seeded. Positive clones in Cell ELISA evaluation were evaluated for their reactivity to HTS015C or hCX3CR1-expressing Ba/F3 cells with a flow cytometer using their culture supernatants or antibodies purified therefrom.

At 1 to 3 days before measurement, the Eurofins cells were seeded in 384-well CellBIND plates (Corning) and cultured at 37°C. The Eurofins cells were cultured in DMEM high Glucose medium (Nacalai Tesque, Inc., Japan) supplemented with 10% FBS (HyClone), 1% MEM NEAA (Gibco), 10 mM Hepes (Gibco) and 1% Penicillin-Streptomycin (Nacalai Tesque, Inc., Japan). The culture supernatants of the Eurofins cells were removed, and the culture supernatants of the hybridomas were each added in a volume of 20 µL per well and allowed to stand at 4°C for 1 hour. The cells were washed with phosphate-buffered saline (PBS), and an HRP-labeled secondary antibody diluted with a blocking buffer (PBS containing 1% bovine serum albumin; 1% BSA/PBS) was added in a volume of 20 µL per well and allowed to stand at 4°C for 1 hour. After the cells were washed with PBS, TMB (Nacalai Tesque, Inc., Japan) was added as a chromogenic substrate solution to cause color development, and after 15 minutes, 1 N sulfuric acid was added to stop the reaction, followed by measurement of absorbance at 450 nm.

The above Cell ELISA positive clones were further evaluated for their reactivity to HTS015C or hCX3CR1-expressing Ba/F3 cells with a flow cytometer using their culture supernatants or antibodies purified therefrom. The purified antibodies of the positive clones were prepared mainly using rProtein A Sepharose Fast Flow.

HTS015C (negative control: HTSCHEM-1) or hCX3CR1-expressing Ba/F3 cells (negative control: Ba/F3 cells transfected with an empty vector) were cultured at 37.0°C in the presence of 5% CO₂. The Ba/F3 cells were cultured in RPMI Medium 1640 (SIGMA) supplemented with 10% FBS (HyClone), 1 ng/mL mouse IL-3 (R&D) and 1% Penicillin-Streptomycin (Nacalai Tesque, Inc., Japan). The Eurofins cells, which are adherent cells, were collected with an enzyme-free cell dissociation solution (Thermo Fisher Scientific). The cells were dispensed into 96-well plates, followed by centrifugation to remove the supernatants. Then, the culture supernatants of the hybridomas or their purified antibodies diluted with 1% BSA/PBS were each added in a volume of 50 µL per well, and the cells were suspended and allowed to stand at 4°C for 30 minutes. After the cells were washed with 1% BSA/PBS, a PE-labeled secondary antibody diluted with 1% BSA/PBS was added in a volume of 50 µL per well, and the cells were suspended and allowed to stand at 4°C for 30 minutes under light-shielded conditions. After the cells were washed with 1% BSA/PBS, a dead cell detection reagent (7-AAD) diluted with 1% BSA/PBS was added, and the culture supernatants of the hybridomas or antibodies purified therefrom were measured for their reactivity to hCX3CR1-expressing cells with a FACS Canto II (Becton Dickinson).

As a result of screening about 30,000 hybridoma clones, 46 clones with specific reactivity to hCX3CR1-expressing cells were selected as positive clones.

### 1-4-2. Screening based on cell migration (chemotaxis) inhibitory activity

Clones with binding activity to hCX3CR1-expressing cells were evaluated for their inhibitory activity against the migration of hCX3CR1-expressing Ba/F3 cells toward fractalkine (also referred to as FKN or CX3CL1), which is a ligand of hCX3CR1.

Cell chemotaxis was measured by Boyden chamber assay. hCX3CR1-expressing Ba/F3 cells were seeded in the upper chambers of a HTS Transwell 96-well plate, 5 µm (Corning), and a medium containing human FKN (PeproTech) was added to the lower chambers. The migrated cells were measured by CellTiter-Glo Luminescent Cell Viability Assay (Promega).

After hCX3CR1-expressing cells were adjusted to 5.33 × 10⁵ cells per 100 µL with a 1% FBS-containing culture medium and cultured at 37°C for 3 to 6 hours, the purified antibodies were each added thereto, followed by culture for an additional 1 hour. The upper chambers were charged with the cell suspensions (finally 75 µL, 2 × 10⁵ cells) and the lower chambers were charged with a 1% FBS-containing culture medium alone or a 10 ng/mL human fractalkine-containing culture medium, followed by overnight culture at 37°C. After culture, the upper chambers were removed by being gently shaken, and the cultured solutions (100 µL) containing the migrated cells in the lower chambers were each mixed with an equal volume of CellTiter-Glo and allowed to stand at room temperature for 10 minutes, followed by measurement of luminescence with an Enspire Alpha Plate Reader (Perkin Elmer).

As a result of evaluating the above 46 clones for their inhibitory activity against the migration of hCX3CR1-expressing Ba/F3 cells, a clone (m1F8) with nearly 100% cell migration inhibitory activity was selected. This clone is only one functional clone screened starting from as many as about 30,000 hybridoma clones, as mentioned in 1-4-1 above, and is therefore extremely difficult to obtain because it cannot be easily obtained by trial and error efforts normally available to those skilled in the art.

### [Example 2]

### 2. Sequence analysis of antibody

The m1F8 antibody thus obtained was analyzed for the sequences of its immunoglobulin gene variable regions.

The m1F8 hybridoma cells were expanded, followed by mRNA extraction, 5' RACE reverse transcription, PCR amplification of antibody variable region sequences, cloning, and sequence analysis by Sanger sequencing (using the service of GENEWIZ).

Amino acid sequences were determined for CDR regions as well as the heavy chain variable region (VH) and the light chain variable region (VL) in the resulting antibody (m1F8 antibody) sequences in accordance with the method of Kabat et al. (Sequences of Proteins of Immunological Interest, Fifth Edition, NIH Publication No. 91-3242, US, Department of Health and Human Services, 1991).

### [Example 3]

### 3. Activity evaluation of m1F8 antibody

### 3-1. Binding activity to hCX3CR1-expressing Ba/F3

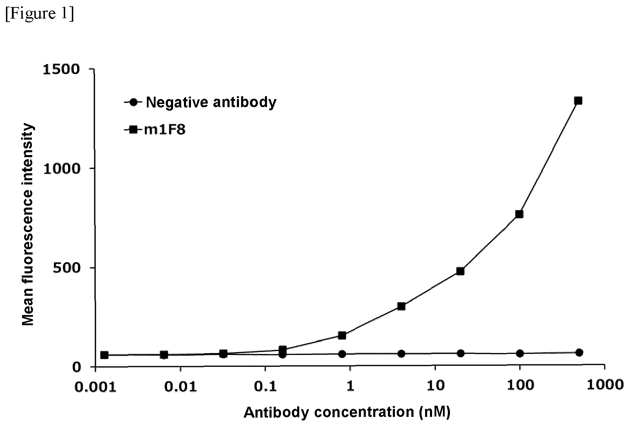

The m1F8 antibody or a negative antibody was adjusted to 9 concentrations in total at a 5-fold common ratio up to the highest concentration of 500 nM, and the binding activity of the m1F8 antibody to hCX3CR1-expressing Ba/F3 cells was measured by flow cytometry. The results obtained are shown in Figure 1. As a negative antibody, a mouse IgG2a isotype control antibody was used. Procedures for flow cytometry were performed in the same manner as shown in 1-4-1 in Example 1. As shown in Figure 1, the negative antibody showed no increase in fluorescence intensity even when the antibody concentration was increased, and no binding was observed to hCX3CR1-expressing Ba/F3 cells. On the other hand, fluorescence intensity in the m1F8 antibody was increased in an antibody concentration-dependent manner, and the m1F8 antibody showed clear reactivity to hCX3CR1-expressing Ba/F3 cells although it did not reach the saturation level at the adjusted antibody concentrations. Moreover, this reactivity was not substantially observed in Ba/F3 cells transfected with an empty vector; and hence this binding was deemed to be a hCX3CR1 expression-dependent specific reaction.

### 3-2. Cell migration inhibitory activity against hCX3CR1-expressing Ba/F3

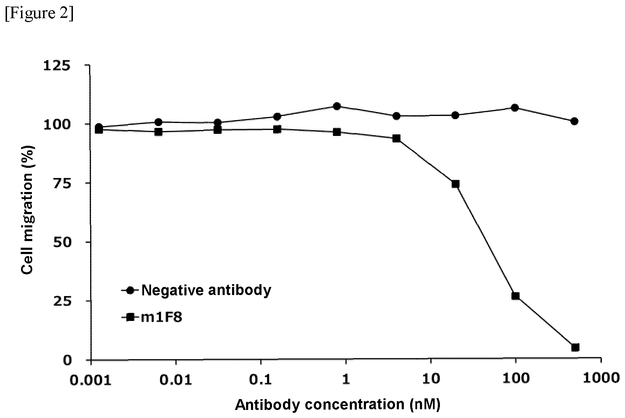

The m1F8 antibody or a negative antibody was adjusted to 9 concentrations in total at a 5-fold common ratio up to the highest concentration of 500 nM, and measured for its inhibitory activity against the fractalkine-dependent cell migration of hCX3CR1-expressing Ba/F3 cells. The results obtained are shown in Figure 2. As a negative antibody, a mouse IgG2a isotype control antibody was used. Procedures for the cell migration test were performed in the same manner as shown in 1-4-2 in Example 1. As shown in Figure 2, the negative antibody showed no inhibition against cell migration even when the antibody concentration was increased. On the other hand, the m1F8 antibody showed 95% or higher inhibitory activity at 500 nM against the FKN-dependent cell migration of hCX3CR1-expressing Ba/F3 cells.

### [Example 4]

### 4. Cross-reactivity of m1F8 antibody to other animal species-derived CX3CR1

The results of m1F8 and various antibodies measured for their species cross-reactivity to CX3CR1 (binding reactivity to human, cynomolgus monkey, mouse and rat CX3CR1-expressing Ba/F3 cells) are shown in Table 1 below. Based on cynomolgus monkey (Uniprot registration number: A0A2K5VJQ4) and rat (P35411), gene synthesis was conducted to obtain DNAs each comprising a Kozak translation initiation sequence inserted at the 5'-terminal end and a translation termination codon inserted on the 3'-terminal side (using the service of GENEWIZ). Using an In-Fusion HD Cloning Kit, the synthesized DNAs were each inserted into a pEF6/V5-His TOPO vector cleaved with restriction enzymes (BamHI and NotI) to obtain various CX3CR1 expression vectors (cynomolgus monkey: cCX3CR1_pEF6/V5-His, rat: rCX3CR1_pEF6/V5-His). In the same manner as used in the preparation of hCX3CR1-expressing Ba/F3 cells (1-1), the various CX3CR1 expression vectors were introduced into Ba/F3 cells through electroporation, and the cells were cultured in a blasticidin-containing selective medium and subjected to a flow cytometer to separate CX3CR1-expressing cells, which were then used as stable expression cell lines in the cross-reactivity test. Procedures for flow cytometry were performed in the same manner as shown in 1-4-1 in Example 1. As positive antibodies, an equivalent to 66B02-mFc (Boehringer Ingelheim), which had been prepared by the inventors of the present invention (i.e., a mFc-fused protein of anti-human CX3CR1-Nanobody), and commercially available anti-mouse CX3CR1 antibody SA011F11 (Biolegend) were used. The reactivity (mean fluorescence intensity) measured under conditions where a secondary antibody was used alone in the presence of the cells was used as a background value for subtraction correction, and the reactivity to hCX3CR1-expressing Ba/F3 cells or cCX3CR1-expressing Ba/F3 cells in 66B02-mFc at 200 nM was set to 100%, or the reactivity to mCX3CR1-expressing Ba/F3 cells or rCX3CR1-expressing Ba/F3 cells in SA011F11 at the same concentration was set to 100%, whereby the % reactivity of the m1F8 antibody at the same concentration was calculated and used for determination of cross-reactivity. In the results shown in Table 1, the relative value compared to the positive antibody at 200 nM was expressed as follows: "+++" for greater than 70%, "++" for 40% to 70%, "+" for 10% to less than 40%, and "-" for less than 10%.

### [Table 1]

**Table 1 Species cross-reactivity of m1F8 antibody**

| | SA011F11 | 66B02-mFc | m1F8 |
|---|---|---|---|
| Human | - | +++ | ++ |
| Cynomolgus monkey | - | +++ | +++ |
| Mouse | +++ | - | - |
| Rat | +++ | - | - |

| | | | |
|---|---|---|---|
| Relative value compared to positive antibody: +++; >70% ++; 40-70% +; 10-40% -; <10% | | | |

As shown in Table 1, SA011F11 was not reactive to human and cynomolgus monkey CX3CR1, and was reactive to mouse and rat CX3CR1. 66B02-mFc and m1F8 were both reactive to human and cynomolgus monkey CX3CR1, and were not reactive to mouse and rat CX3CR1. In view of the foregoing, m1F8 showed cross-reactivity to primate CX3CR1, but no cross-reactivity to rodent CX3CR1.

### [Example 5]

### 5. Epitope analysis of m1F8 antibody

### 5-1. Binding domain analysis of m1F8 antibody

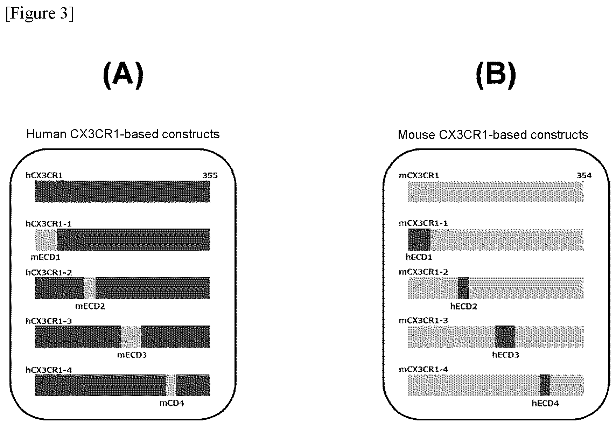

Figure 3 shows schematic views of chimeric CX3CR1 expression constructs prepared for epitope analysis, in which extracellular domains were exchanged between human and mouse CX3CR1. Gene sequences were synthesized such that four extracellular domains present in the CX3CR1 protein sequence were exchanged one by one between human and mouse CX3CR1 (using the service of GENEWIZ). Using an In-Fusion HD Cloning Kit, the synthesized DNAs were each inserted into a pEF6/V5-His **TOPO** vector cleaved with restriction enzymes (BamHI and NotI) to prepare 8 chimeric CX3CR1 expression vectors in total. In the same manner as used in the preparation of hCX3CR1-expressing Ba/F3 cells (1-1), the various CX3CR1 expression vectors were introduced into Ba/F3 cells through electroporation, and the cells were cultured in a blasticidin-containing selective medium and subjected to a flow cytometer to separate CX3CR1-expressing cells, which were then used as stable expression cell lines in binding domain analysis.

The results of m1F8 and various antibodies measured for their binding activity to chimeric CX3CR1-expressing Ba/F3 cells are shown in Tables 2 and 3 below. Procedures for flow cytometry were performed in the same manner as shown in 1-4-1 in Example 1. As positive antibodies, 66B02-mFc equivalent and SA011F11 were used. As a negative antibody, a mouse IgG2a isotype control antibody was used. 66B02-mFc was confirmed to show clear reactivity to the expression cell lines having hCX3CR1 extracellular domain 1 (hCX3CR1, hCX3CR1-2, -3 and -4, and mCX3CR1-1), while SA011F11 was confirmed to show clear reactivity to the expression cell lines having mCX3CR1 extracellular domain 1 (mCX3CR1, hCX3CR1-1, and mCX3CR1-2, -3 and -4). Based on this result, the reactivity (mean fluorescence intensity) of the control antibody at 200 nM to each cell line was used for subtraction correction, and the reactivity to each expression cell line having hCX3CR1 extracellular domain 1 in 66B02-mFc at the same concentration was set to 100%, or the reactivity to each expression cell line having mCX3CR1 extracellular domain 1 in SA011F11 at the same concentration was set to 100%, whereby the % reactivity of the m1F8 antibody at the same concentration was calculated and used to identify binding domains. In the results shown in Tables 2 and 3, the relative value compared to the positive antibody at 200 nM was expressed as follows: "+++" for greater than 70%, "++" for 40% to 70%, "+" for 10% to less than 40%, and "-" for less than 10%.

### [Table 2]

**Table 2 Epitope analysis**

| | SA011F11 | 66B02-mFc | m1F8 | K0124E1 |
|---|---|---|---|---|
| hCX3CR1 | - | +++ | +++ | ++ |
| hCX3CR1-1 | +++ | - | + | - |
| hCX3CR1-2 | - | +++ | +++ | +++ |
| hCX3CR1-3 | - | +++ | +++ | ++ |
| hCX3CR1-4 | - | +++ | +++ | +++ |

| | | | | |
|---|---|---|---|---|
| Relative value compared to positive antibody: +++; >70% ++; 40-70% +; 10-40% -; <10% | | | | |

### [Table 3]

**Table 3 Epitope analysis**

| | SA011F11 | 66B02-mFc | m1F8 | K0124E1 |
|---|---|---|---|---|
| mCX3CR1 | +++ | - | - | - |
| mCX3CR1-1 | - | +++ | ++ | +++ |
| mCX3CR1-2 | +++ | - | - | - |
| mCX3CR1-3 | +++ | - | - | - |
| mCX3CR1-4 | +++ | - | + | - |

| | | | | |
|---|---|---|---|---|
| Relative value compared to positive antibody: +++; >70% ++; 40-70% +; 10-40% -; <10% | | | | |

As shown in Tables 2 and 3, m1F8 showed reactivity to the expression cell lines having hCX3CR1 extracellular domain 1 (hCX3CR1, hCX3CR1-2, -3 and -4, and mCX3CR1-1), and also showed some reactivity to the expression cell lines not having hCX3CR1 extracellular domain 1 (hCX3CR1-1 and mCX3CR1-4). On the other hand, another commercially available anti-hCX3CR1 antibody K0124E1 (Biolegend) showed reactivity only to the expression cell lines having hCX3CR1 extracellular domain 1, as in the case of 66B02-mFc. The foregoing results strongly suggested a possibility that the m1F8 antibody would recognize the conformational structure of CX3CR1 when compared to other antibodies.

### [Example 6]

### 6. Antibody binning

### 6-1. Binning of 1F8 and control antibodies

The results of epitope binning for 1F8 and various antibodies using human CX3CR1 (hCX3CR1)-expressing Ba/F3 cells are shown in Table 4.

The competitive antibodies (10 µM) indicated in the left column of Table 4 and the detection antibodies (100 nM) indicated in the upper row of Table 4 were added to hCX3CR1-expressing Ba/F3 cells, and the cells were suspended and allowed to stand at 4°C for 30 minutes under light-shielded conditions. The subsequent procedures for flow cytometry were performed in the same manner as shown in 1-4-1 in Example 1. m/h1F8 represents a chimeric antibody having Fab of mouse origin and Fc of human origin. As control antibodies, 66B02-mFc equivalent, 306D-hFc equivalent (i.e., a hFc-fused protein of anti-hCX3CR1-Nanobody), and K0124E1 were used. As a negative antibody, Synagis (palivizumab) equivalent (herein denoted as "Synagis"), which is a humanized IgG1 antibody, was used. The reactivity (mean fluorescence intensity) measured under conditions where a secondary antibody was used alone in the presence of the cells was used as a background value for subtraction correction, and the reactivity of each detection antibody when using Synagis as a competitive antibody was set to 100%, whereby the % reactivity of each detection antibody in the presence of each competitive antibody was calculated and used to determine binding competition. The relative value compared to the competitive negative antibody was expressed as follows: "+++" for greater than 70%, "++" for 40% to 70%, "+" for 10% to less than 40%, and "-" for less than 10%.

### [Table 4]

**Table 4**

| | | Detection antibody | | |
|---|---|---|---|---|
| | | m1F8 | 66B02-mFc | K0124E1 |
| Competitive antibody | Negative antibody | +++ | +++ | +++ |
| | m/h1F8 | - | +++ | +++ |
| | 306D-hFc | +++ | - | - |

| | | | | |
|---|---|---|---|---|
| Relative value compared to negative competitive antibody: +++; >70% ++; 40-70% +; 10-40% -; <10% | | | | |

As shown in Table 4, the 1F8 antibodies very strongly competed with each other, and did not significantly compete with the control antibodies 66B02-mFc, 306D-hFc and K0124E1, whereas the control antibody 306D-hFc was shown to very strongly compete with 66B02-mFc and K0124E1 (a commercially available antibody).

### 6-2. Activity enhancement of m1F8 antibody

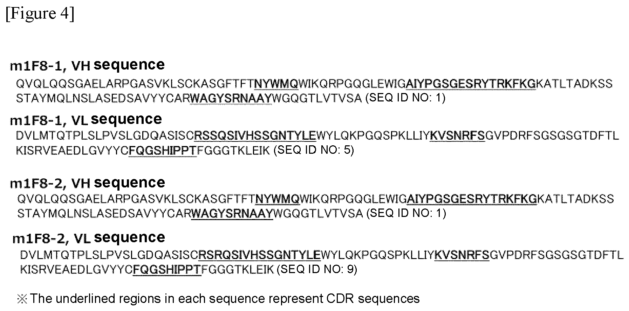

The results of the previous examples infer that when compared to known anti-CX3CR1 antibodies, the m1F8 antibody is more likely to greatly differ in terms of properties, particularly epitope recognition, but is very low in binding activity to CX3CR1 and in inhibitory activity against CX3CR1. To overcome this drawback, on the basis of the findings about the activity enhancement of antibody sequences using ADLib^{®}, mutations were artificially introduced into the CDR sequences of the m1F8 antibody to obtain a plurality of activity-enhanced m1F8 antibodies (m1F8-1 and m1F8-2). Figure 4 shows the amino acid sequences of the heavy chain variable region (VH) and the light chain variable region (VL) of m1F8-1 and m1F8-2 thus obtained. In Figure 4, the underlined regions in each amino acid sequence represent CDRs.

Table 5 shows binding activity to hCX3CR1-expressing Ba/F3 cells (EC50) and inhibitory activity against fractalkine-dependent cell migration (IC50) measured for the m1F8 antibody, two activity-enhanced m1F8 antibodies (m1F8-1 and m1F8-2) and anti-hCX3CR1-Nanobodies.

### [Table 5]

**Table 5**

| | Cell binding activity (EC50, nM) | Cell migration inhibitory activity (IC50, nM) |
|---|---|---|
| m1F8 | 199 | 55.2 |
| m1F8-1 | 6.69 | 5.32 |
| m1F8-2 | 2.67 | 2.96 |
| 66B02-mFc | 0.145 | - |
| BII00313 | - | 1.54 |

As shown in Table 5, as a result of activity enhancement, the activity-enhanced m1F8 antibodies (m1F8-1 and m1F8-2) achieved up to a 70-fold or more improvement in binding activity and a 15-fold or more improvement in inhibitory activity when compared to the original m1F8 antibody. However, these activities were still higher in the anti-hCX3CR1-Nanobodies than in these activity-enhanced m1F8 antibodies.

### 6-3. Activity-enhanced 1F8 antibody binning

The results of epitope binning for activity-enhanced 1F8 antibodies (1F8-1 and 1F8-2) and anti-hCX3CR1-Nanobodies using hCX3CR1-expressing Ba/F3 cells are shown in Table 6. The same procedures as shown in 6-1 above were repeated.

### [Table 6]

**Table 6**

| | | Detection antibody | | |
|---|---|---|---|---|
| | | m1F8-1 | m1F8-2 | 66B02-mFc |
| Competitive antibody | Negative antibody | +++ | +++ | +++ |
| | m/h1F8-1 | - | - | +++ |
| | m/h1F8-2 | - | - | +++ |
| | 306D-hFc | +++ | +++ | - |

| | | | | |
|---|---|---|---|---|
| Relative value compared to negative competitive antibody: +++; >70% ++; 40-70% +; 10-40% -; <10% | | | | |

As shown in Table 6, very strong competition was observed between 1F8 antibodies or between Nanobodies, whereas no significant competition was observed between 1F8 and Nanobody.

The results of Tables 4 and 6 suggest that 1F8 and other antibodies (known antibodies) have different epitopes.

### [Example 7]

### 7. Preparation of human CX3CR1-KI mouse late-onset EAE model and results of BII00313 administration test

### 7-1. Preparation of genetically modified mice

The mice used were all at 6 to 8 weeks of age and were kept under conditions free from specific pathogens. For preparation of NR4A2-deficient mice (NR4A2-cKO), a targeting vector comprising the NR4A2 gene sandwiched between loxp sequences was used to establish NR4A2^{fl/fl} mice. Namely, the NR4A2 transgene sandwiched between loxp sequences was introduced by microinjection into C57BL/6 embryonic stem cells. The established line was bred with C57BL/6 FLPe mice (Riken BioResource Research Center, Japan) to remove the neomycin cassette, and the resulting lines were crossed with each other to prepare homozygous NR4A2^{fl/fl} C57BL/6 mice. The resulting mice were crossed with C57BL/6 CD4-Cre (Taconic Biosciences) to establish CD4-specific NR4A2-cKO C57BL/6 mice (C57BL/6 Cre-CD4/NR4A2^{fl/fl} mice = NR4A2-cKO mice). For preparation of mice whose mouse CX3CR1 was replaced with human CX3CR1 (human CX3CR1-KI mice), the mouse CX3CR1 gene was replaced with the human CX3CR1 gene by genome editing to establish human CX3CR1-KI mice. Namely, a Cas9 expression vector was first constructed to comprise a guide RNA sequence targeting the exon 2 protein encoding region in the target gene (Cx3cr1 gene, Gene ID: 13051). Then, a homologous recombination vector was constructed to comprise an artificially synthesized sequence comprising the knock-in sequence (CX3CR1 gene, Gene ID: 1524, NM_001337.3, CDS region, 1068 bp) and a homologous region cloned from the genome of the C57BL/6N-derived RENKA line.

Then, the guide Cas9 vector, the homologous recombination vector and a transient drug (Puromycin) resistance gene expression vector were introduced by electroporation into ES cells (C57BL/6N-derived RENKA line), and colonies of ES cell clones formed upon drug selection culture were isolated. Then, ES cell clones confirmed to have undergone homologous recombination were used to prepare chimeric embryos by aggregation techniques using ICR recipient embryos, and the resulting chimeric embryos were transplanted into recipient mice to prepare chimeric mice. The germ-line chimeric mice were naturally crossed with wild-type mice (C57BL/6N) to prepare first-generation mice (KI heterozygous mice), and the KI heterozygous mice were crossed with each other to prepare KI homozygous mice (human CX3CR1-KI mice). For preparation of NR4A2-cKO/human CX3CR1-KI mice, the NR4A2-cKO mice were crossed with the human CX3CR1-KI mice to finally obtain CD4-Cre^{+/-} /NR4A2^{fl/fl}/CX3CR1^{human/human} mice, which were used as KI late-onset EAE test mice.

### 7-2. EAE induction (monophasic EAE)

A PBS solution containing 100 µg of a peptide corresponding to MOG 35-55 residues (synthesized in Toray Research Center, Japan; hereinafter also referred to as an "MOG peptide") and complete Freund's adjuvant (CFA) containing 1 mg of killed *Mycobacterium tuberculosis* H37Ra (Difco, Kansas, USA) were mixed in equal volumes and emulsified with a sonicator to prepare an MOG emulsion. The resulting MOG emulsion was used to immunize NR4A2-cKO/human CX3CR1-KI mice by subcutaneous injection at one or two sites on the back. Further, on Day 0 and Day 2 after immunization, the mice were intraperitoneally injected with 200 µL of a PBS solution containing 200 ng per mouse of pertussis toxin (List Biological Laboratories,

### USA).

### 7-3. Results of antibody administration test in KI mouse late-onset EAE model (BII00313)

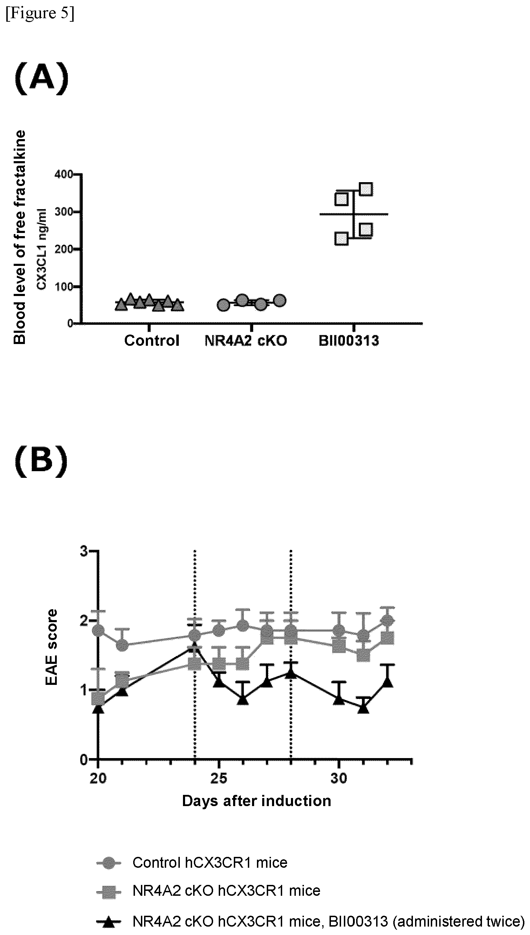

Human CX3CR1-KI mice induced to develop monophasic EAE were administered with PBS on Day 24 and Day 28 after EAE induction (denoted as Control in Figure 5(A) and (B)). Likewise, NR4A2-cKO/human CX3CR1-KI mice were administered with PBS or 200 µg of anti-CX3CR1-Nanobody (BII00313) on Day 24 and Day 28 after EAE induction (denoted as NR4A2cKO, BII00313 in Figure 5(A) and (B)). In accordance with the evaluation criteria for EAE shown below, EAE pathology was evaluated for each mouse.

### <Evaluation criteria for EAE>

0: no clinical sign
1: partial tail paralysis
2: flaccid tail
3: partial hind limb paralysis
4: complete hind limb paralysis
5: hind limb and forelimb paralysis

Moreover, to confirm that the antibody inhibited ligand-receptor binding, free fractalkine in blood was quantified. On the final day of the test, a triple anesthetic mixture (medetomidine, midazolam, butorphanol) was intraperitoneally administered to anesthetize the mice, and serum collected from each mouse by neck incision was used to quantify free fractalkine in blood with a commercially available ELISA kit (R&D systems, MCX310).

As shown in Figure 5(A), the blood level of free fractalkine was significantly increased in the BII00313-receiving group when compared to the control group and the NR4A2cKO group. This change would be because BII00313 administration inhibited the binding between CX3CR1 and fractalkine, which in turn caused an increase in the blood level of free fractalkine. As shown in Figure 5(B), the BII00313-receving group showed a weak improvement in EAE pathology when compared to the PBS-receiving group.

### [Example 8]

### 8. Results of antibody administration test in human CX3CR1-KI mouse late-onset EAE model (comparison of m1F8 antibodies; comparison between m1F8-1 and m1F8-2)

NR4A2-cKO/human CX3CR1-KI mice induced to develop monophasic EAE in the same manner as shown in Example 7 were administered with 200 µg of anti-hCX3CR1 antibody (m1F8-1 or m1F8-2) or an isotype antibody thereof (mIgG2a) on Day 24 and Day 28 after EAE induction. In the same manner as shown in Example 7, EAE pathology was evaluated for each mouse. Moreover, blood fractalkine was measured.

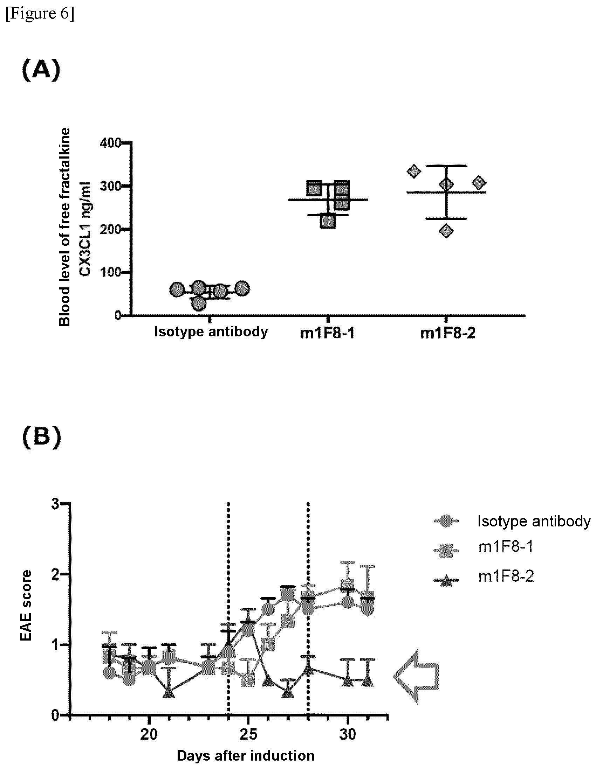

As shown in Figure 6(A), the blood level of free fractalkine was significantly increased in the m1F8 antibody-receiving groups when compared to the isotype antibody-receiving group. In addition, there was no significant difference between m1F8 antibodies (i.e., between m1F8-1 antibody and m1F8-2 antibody). On the other hand, as shown in Figure 6(B), only the m1F8-2 antibody-receiving group showed clearly improved EAE pathology. The m1F8-1 antibody-receiving group showed a slight improvement in EAE score after the first administration, but then behaved close to the EAE score of the isotype antibody-receiving group. Under circumstances where there are antibodies, like the m1F8-1 antibody, whose good results of *in vitro* functional activity (increase in the blood level of free fractalkine) are not always clearly related to *in vivo* functional evaluation (improvement in EAE pathology), the m1F8-2 antibody not only shows good results of *in vitro* functional activity, but is also excellent in *in vivo* functional evaluation. An anti-hCX3CR1 antibody functionally excellent both *in vitro* and *in vivo,* like the m1F8-2 antibody, is extremely difficult to obtain because it cannot be easily obtained by trial and error efforts normally available to those skilled in the art.

### [Example 9]

### 9. Results of antibody administration test in human CX3CR1-KI mouse late-onset EAE model (comparison between BII00313 and m1F8-2 antibody)

### 9-1. EAE score

NR4A2-cKO/human CX3CR1-KI mice induced to develop monophasic EAE in the same manner as shown in Example 7 were administered with 200 µg of BII00313, m1F8-2 or an isotype antibody thereof (mIgG2a) on Day 24 and Day 27 after EAE induction. In the same manner as shown in Example 7, EAE pathology was evaluated for each mouse.

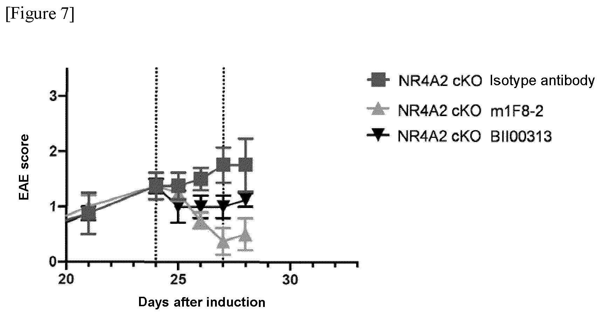

As shown in Figure 7, the m1F8-2-receiving group particularly showed strongly improved EAE pathology.

9-2. Evaluation of the number of cells infiltrated into the central nervous system

NR4A2-cKO/human CX3CR1-KI mice induced to develop monophasic EAE in the same manner as shown in Example 7 were administered with 200 µg of BII00313, m1F8-2 or an isotype antibody thereof on Day 24, Day 28 and Day 31 after EAE induction. On day 32 after EAE induction, the brain and spinal cord were taken from each mouse to evaluate the number of cells infiltrated into the central nervous system. In more detail, 6 mice in each antibody-receiving group were further divided into two groups (pool 1 and pool 2), and their tissues were each cut into small pieces and then further digested at 37°C for 40 minutes in RPMI 1640 medium (Invitrogen) containing 1.4 mg/mL collagenase H and 100 µg/mL DNase (Roche). The resulting tissue homogenates were each passed through a 70 µm cell strainer (GE Healthcare Life Sciences), and leukocyte cells were enriched by using discontinuous Percoll density gradient centrifugation (37%/80%). Then, using a flow cytometer, CD45, TCRβ and CD4-positive T cells infiltrated into the CNS (central nervous system) were separated with a FACS Aria II (BD Cytometry Systems). In Table 7(A), the cells were further cultured for 5 hours in the presence of 5 ng/mL phorbol myristate acetate, 500 ng/mL ionomycin (Sigma) and GolgiPlug (BD Biosciences), fixed and membrane-permeabilized with a Foxp3/Transcription Factor Staining Buffer Set (eBioscience), and then stained with an anti-IL-17 antibody and an anti-IFN-y antibody, followed by analysis with a FACS Canto II (BD Cytometry Systems). In Table 7(B), the separated CD4 positive T cells were pre-stained with an anti-CX3CR1 antibody, fixed and membrane-permeabilized with a Foxp3/Transcription Factor Staining Buffer Set (eBioscience), and then further stained with an anti-Granzyme B antibody, followed by analysis. Among the CD45, TCRβ and CD4-positive cells, the ratio of IL-17 negative and IFN-y positive cells (A) and the ratio of Granzyme B and CX3CR1 double-positive cells (B) are shown in Table 7. For separation and detection of the cells, the following antibodies were used: an anti-CD45 antibody (Biolegend), an anti-TCRβ antibody (Biolegend), an anti-CD4 antibody (Biolegend), an anti-IL-17 antibody (Biolegend), an anti-IFN-y antibody (Biolegend), an anti-CX3CR1 antibody (Biolegend), and an anti-Granzyme B antibody (Biolegend).

### [Table 7]

**Table 7 Evaluation of infiltrated cells in the central nervous system (flow cytometry)**

| (A) | | | | | | |
|---|---|---|---|---|---|---|
| Antibody administered | Isotype | | BII00313 | | m1F8-2 | |
| Pool No. | 1 | 2 | 1 | 2 | 1 | 2 |
| Ratio of IL-17 negative and IFN-y positive cells (%) | 24.4 | 29.1 | 22.3 | 21.0 | 8.62 | 6.97 |

| (B) | | | | | | |
|---|---|---|---|---|---|---|
| Antibody administered | Isotype | | BII00313 | | m1F8-2 | |
| Pool No. | 1 | 2 | 1 | 2 | 1 | 2 |
| Ratio of GZMB positive cells (%) | 28.8 | 39.4 | 14.1 | 32.6 | 16.1 | 10.2 |
| Ratio of GZMB and CX3CR1 double-positive cells (%) | 23.7 | 35.3 | 12.6 | 28.5 | 14.4 | 8.09 |

As shown in Table 7A, the m1F8-2 antibody-receiving group showed a decrease in IL-17 negative and IFN-y positive cells. In addition, as shown in Table 7B, the m1F8-2 antibody-receiving group showed decreases in Granzyme B (GZMB) positive cells (upper) and GZMB and CX3CR1 double-positive cells (lower). CD4 and IFN-γ- or CD4 and GZMB-positive cells are interpreted to mean cytotoxic Th cells (cytotoxic helper T cells), and a series of results suggests a possibility that the m1F8-2 antibody would act on CX3CR1 to reduce cytotoxic Th cells in the central nervous system, i.e., would inhibit or suppress the infiltration of cytotoxic Th cells into the CNS, thereby resulting in strongly improved EAE pathology.

### [Example 10]

### 10. Humanized 1F8 antibodies (h1F8-2-1, h1F8-2-2, h1F8-2-3 and h1F8-2-4)

### 10-1. Design for humanization of 1F8 antibody

The sequences of the m1F8-2 antibody confirmed to have efficacy *in vitro* and *in vivo* were humanized. The sequences of antibody variable regions were humanized by CDR grafting. The design of humanized sequences was accomplished on the basis of procedures described in a known article (Tsurushita et al., Design of humanized antibodies: From anti-Tac to Zenapax. Methods, 36:69-83, 2005).

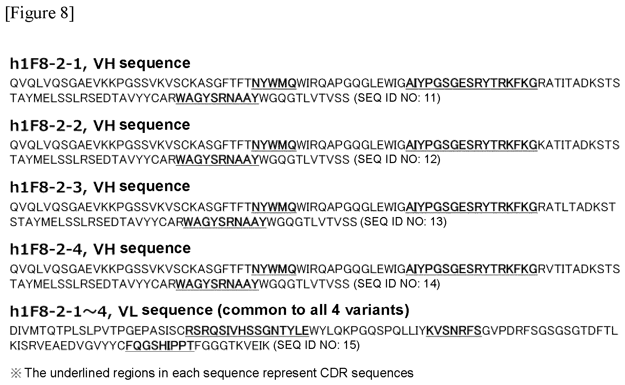

First, a three-dimensional molecular model was prepared for mouse antibody in a standard manner. Then, this molecular model was used to estimate residues considered to be important for CDR structure formation and residues considered to be essential for reaction with the antigen in the amino acid sequences of framework regions. In parallel, the cDNA sequence databases of human antibody heavy and light chain variable regions were searched for sequences highly homologous to the heavy and light chain variable regions of anti-hCX3CR1 antibody. Then, the sequences of framework portions in the searched human antibody sequences were linked to the CDR sequences of each anti-hCX3CR1 antibody, and the thus designed sequences were further grafted with a sequence of residues considered to be essential for CDR structure formation or for reaction with the antigen to thereby design humanized antibody sequences. The designed sequences are shown in Figure 8. CDR sequences in the humanized antibody sequences are the same as those in the original mouse antibody, and are the same sequences as shown in the SEQ ID NOs mentioned above (i.e., SEQ ID NOs: 2 to 4 as well as SEQ ID NOs: 10, 7 and 8). In Figure 8, the underlined regions in each amino acid sequence represent CDRs.

Four VH sequences and one VL sequence were designed; and hence 4 types of humanized 1F8 antibodies (h1F8-2-1, h1F8-2-2, h1F8-2-3 and h1F8-2-4) were obtained as possible combinations of humanized sequences. These humanized antibodies were evaluated for their binding activity to hCX3CR1-expressing Eurofins cells (EC50) and their inhibitory activity against the fractalkine-dependent cell migration of human CX3CR1-expressing Ba/F3 cells (IC50). The results obtained are shown in Table 8.

### [Table 8]

**Table 8 EC50 and IC50 in humanized 1F8 antibodies**

| | Binding activity (EC50, nM) | Cell migration inhibitory activity (IC50, nM) |
|---|---|---|
| h1F8-2-1 | 4.42 | 2.02 |
| h1F8-2-2 | 3.10 | 1.27 |
| h1F8-2-3 | 4.02 | 1.40 |
| h1F8-2-4 | 4.42 | 2.61 |

As shown in Table 8, the humanized 1F8 antibodies (h1F8-2-1, h1F8-2-2, h1F8-2-3 and h1F8-2-4) were found to have binding activity and inhibitory activity, as in the case of the above m1F8 antibody.

### [Example 11]

### 11. Cross-reactivity of modified 1F8 antibodies to other animal species-derived CX3CR1

### 11-1. Cross-reactivity of activity-enhanced m1F8 antibodies (m1F8-1 and m1F8-2) to other animal species-derived CX3CR1

The results of the m1F8-1 antibody or the m1F8-2 antibody evaluated for its species cross-reactivity to CX3CR1 (binding reactivity to human, cynomolgus monkey, mouse and rat CX3CR1-expressing Ba/F3 cells) are shown in Table 9 below. The same procedures as used in Example 4 were repeated to conduct flow cytometry and quantification to determine cross-reactivity. In the results shown in Table 9, the relative measured value compared to the measured value of the positive antibody at 200 nM was expressed as follows: "+++" for greater than 70%, "++" for 40% to 70%, "+" for 10% to less than 40%, and "-" for less than 10%.

As shown in Table 9, the m1F8-1 antibody or the m1F8-2 antibody was reactive to human and cynomolgus monkey CX3CR1, and was not reactive to mouse and rat CX3CR1. In view of the foregoing, the m1F8-1 antibody and the m1F8-2 antibody showed cross-reactivity to primate CX3CR1, but no cross-reactivity to rodent CX3CR1, as in the case of the original m1F8 antibody before activity enhancement.

### 11-2. Cross-reactivity of humanized 1F8 antibody (h1F8-2-2) to other animal species-derived CX3CR1

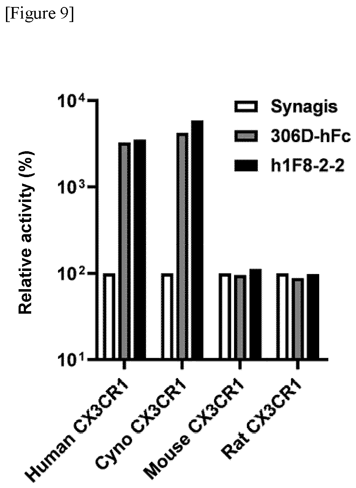

The results of the h1F8-2-2 antibody or 306D-hFc evaluated for its species cross-reactivity to CX3CR1 (binding reactivity to human (Human), cynomolgus monkey (Cyno), mouse (Mouse) and rat (Rat) CX3CR1-expressing Ba/F3 cells) are shown in Figure 9. The same procedures as used in Example 4 were repeated to conduct flow cytometry. Data are shown as relative values compared to the mean fluorescence intensity of Synagis used as a negative antibody.

As shown in Figure 9, the h1F8-2-2 antibody and 306D-hFc were reactive to human and cynomolgus monkey CX3CR1, and were not reactive to mouse and rat CX3CR1. In view of the foregoing, the h1F8-2-2 antibody and 306D-hFc showed cross-reactivity to primate CX3CR1, but no cross-reactivity to rodent CX3CR1.

### [Table 9]

**Table 9**

| | m1F8-1 | m1F8-2 |
|---|---|---|
| Human | +++ | +++ |
| Cynomolgus monkey | +++ | +++ |
| Mouse | - | - |
| Rat | - | - |

| | | |
|---|---|---|
| Relative value compared to positive antibody: +++; >70% ++; 40-70% +; 10-40% -; <10% | | |

### [Example 12]

### 12. Epitope analysis of modified 1F8 antibodies

### 12-1. Binding domain analysis of activity-enhanced m1F8 antibodies (m1F8-1 and m1F8-2)

The results of the m1F8-1 antibody or the m1F8-2 antibody evaluated for its binding activity to chimeric CX3CR1-expressing Ba/F3 cells are shown in Table 10 below. The same procedures as used in Example 5 were repeated to conduct flow cytometry and quantification to identify binding domains. In the results shown in Table 10, the relative measured value compared to the measured value of the positive antibody at 200 nM was expressed as follows: "+++" for greater than 70%, "++" for 40% to 70%, "+" for 10% to less than 40%, and "-" for less than 10%.

As shown in Table 10, the m1F8-1 antibody and the m1F8-2 antibody showed reactivity to the expression cell lines having hCX3CR1 extracellular domain 1 (hCX3CR1, hCX3CR1-2, -3 and -4, and mCX3CR1-1), and also showed some reactivity to hCX3CR1-1 not having hCX3CR1 extracellular domain 1. Further, the m1F8-2 antibody also showed some reactivity to mCX3CR1-4 not having hCX3CR1 extracellular domain 1. The foregoing results strongly suggested a possibility that the m1F8-1 antibody and the m1F8-2 antibody would recognize the conformational structure of CX3CR1, as in the case of the original m1F8 antibody before activity enhancement.

### 12-2. Binding domain analysis of humanized 1F8 antibody (h1F8-2-2)

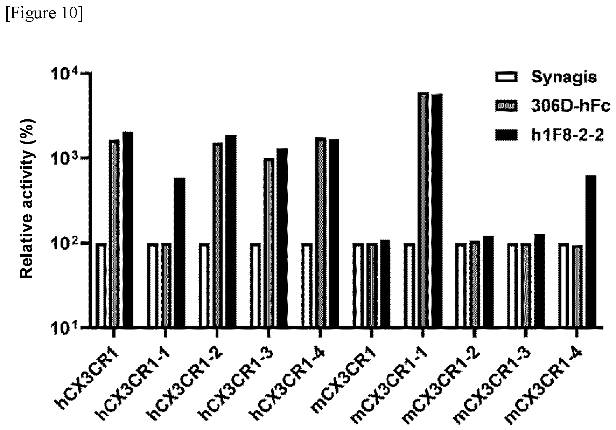

The results of binding domain analysis on the h1F8-2-2 antibody or 306D-hFc are shown in Figure 10. The same procedures as used in Example 5 were repeated to conduct flow cytometry. Data are shown as relative values compared to the mean fluorescence intensity of Synagis used as a negative antibody.

As shown in Figure 10, the h1F8-2-2 antibody showed reactivity to the expression cell lines having hCX3CR1 extracellular domain 1 (hCX3CR1, hCX3CR1-2, -3 and -4, and mCX3CR1-1), and also showed some reactivity to the expression cell lines not having hCX3CR1 extracellular domain 1 (hCX3CR1-1 and mCX3CR1-4). On the other hand, 306D-hFc showed reactivity only to the expression cell lines having hCX3CR1 extracellular domain 1. The foregoing results strongly suggested a possibility that the h1F8-2-2 antibody would also recognize the conformational structure of CX3CR1, as in the case of other 1F8 antibodies.

### [Table 10]

**Table 10**

| | m1F8-1 | m1F8-2 |
|---|---|---|
| hCX3CR1 | +++ | +++ |
| hCX3CR1-1 | ++ | ++ |
| hCX3CR1-2 | +++ | +++ |
| hCX3CR1-3 | +++ | +++ |
| hCX3CR1-4 | +++ | +++ |
| mCX3CR1 | - | - |
| mCX3CR1-1 | +++ | +++ |
| mCX3CR1-2 | - | - |
| mCX3CR1-3 | - | - |
| mCX3CR1-4 | - | + |

| | | |
|---|---|---|
| Relative value compared to positive antibody: +++; >70% ++; 40-70% +; 10-40% -; <10% | | |

### [Example 13]

### 13. Competition test with h1F8-2-2 antibody

The results of a binding competition test on the humanized h1F8-2-2 antibody or anti-hCX3CR1-Nanobody using hCX3CR1-expressing Ba/F3 cells are shown in Table 11. Procedures for the test were performed in the same manner as shown in Example 6.

The competitive antibodies (10 µM) indicated in the left column of Table 11 and the detection antibodies (100 nM) indicated in the upper row of Table 11 were added to hCX3CR1-expressing Ba/F3 cells, and the cells were suspended and allowed to stand at 4°C for 30 minutes under light-shielded conditions. The subsequent procedures for flow cytometry were performed in the same manner as shown in Example 6. The detection antibody h1F8-2-2 (mFc type) represents a chimeric antibody having Fab of the h1F8-2-2 antibody, which is a humanized h1F8 antibody, and mouse Fc. As a negative antibody, Synagis, which is a humanized IgG1 antibody, was used. The reactivity (mean fluorescence intensity) measured under conditions where a secondary antibody was used alone in the presence of the cells was used as a background value for subtraction correction, and the reactivity of each detection antibody when using Synagis as a competitive antibody was set to 100%, whereby the % reactivity of each detection antibody in the presence of each competitive antibody was calculated and used to determine binding competition. The relative value compared to the competitive negative antibody was expressed as follows: "+++" for greater than 70%, "++" for 40% to 70%, "+" for 10% to less than 40%, and "-" for less than 10%.

As shown in Table 11, very strong competition was observed between h1F8-2-2 antibodies or between anti-hCX3CR1-Nanobodies, whereas no significant competition was observed between h1F8-2-2 antibody and Nanobody. This result suggest that the h1F8-2-2 antibody and the anti-hCX3CR1-Nanobodies have different epitopes.

### [Table 11]

**Table 11**

| | | Detection antibody | |
|---|---|---|---|
| | | h1F8-2-2 (mFc type) | 66B02-mFc |
| Competitive antibody | h1F8-2-2 | - | +++ |
| | BII00313 | +++ | - |

| | | | |
|---|---|---|---|
| Relative value compared to negative competitive antibody: +++; >70% ++; 40-70% +; 10-40% -; <10% | | | |

### [Example 14]

### 14. Ca-influx assay on h1F8-2-2 antibody

The h1F8-2-2 antibody was evaluated for its *in vitro* inhibitory activity against hCX3CR1-expressing cells by FKN-induced Ca-influx assay.

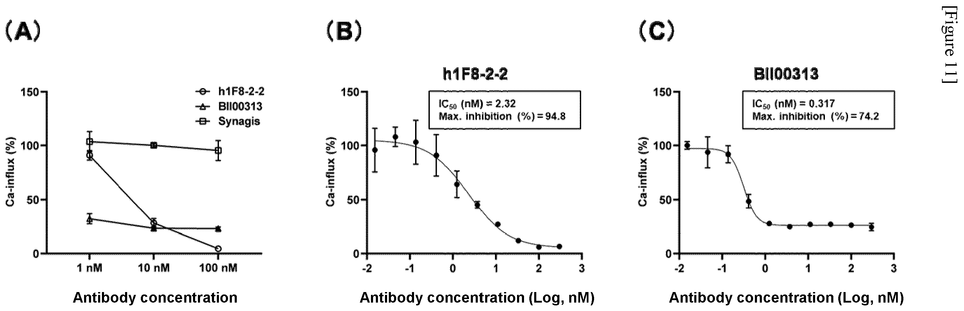

For Ca-influx assay, hCX3CR1-expressing CHO-K1 cells (AequoZen Human Chemokine CX3CR1 Cell Line, CHO-K1 Frozen Cells, Perkin Elmer) were used. The cells were thawed and washed, and then suspended in DMEM/F12 (Gibco) medium containing 5 µM Coelenterazine h (Promega), 0.1% BSA and 1% Penicillin-Streptomycin (Nacalai Tesque, Inc., Japan) and cultured at room temperature for 4 hours. Then, 3 volumes of Coelenterazine h-free culture medium were added, and the cells were suspended and cultured for an additional 30 minutes, and then mixed with an equal volume (25 µL) of the antibody solution in each well of a 96-well white plate (Thermo Fisher Scientific) and cultured for 30 minutes. Human FKN (PeproTech) was added in a volume of 50 µL per well, and luminescence was measured over time using a TriStar2S LB942 Multimode Reader (Berthold Technologies) with Instrument Control and Evaluation software. The final cell counts were 20,000 cells per well, the FKN concentration was 10 nM, and the antibody concentration was used as indicated in Figure 11. The relative luminescence intensity was measured every second for 30 seconds after addition of FKN to the cell suspension, and the maximum value during measurements in the presence of FKN and in the absence of the antibody was set to 100%, whereby the relative value of the maximum value during measurements under each condition was plotted in figures.

Figure 11(A) shows the results measured for Ca-influx in the presence of the h1F8-2-2 antibody or BII00313 (anti-hCX3CR1-Nanobody) at a final concentration of 100, 10 or 1 nM (in a state where the cells, the antibody and the ligand were all added). As a negative antibody, the Synagis antibody was used. When compared to BII00313, the h1F8-2-2 antibody showed low Ca-influx inhibitory activity at low concentration, and showed high inhibitory activity at high concentration.

Figure 11(B) and (C) show the results measured for Ca-influx in the presence of the h1F8-2-2 antibody (Figure 11(B)) or BII00313 (Figure 11(C)) at 10 concentrations in total at a 3-fold common ratio up to the highest concentration of 300 nM. In comparison of IC50, BII00313 showed a lower value than the h1F8-2-2 antibody, whereas the maximum inhibitory activity was higher in h1F8-2-2. The foregoing results indicate that differences between these two antibodies lie not only in their epitopes but also in their activity characteristics.

### [Example 15]

### 15. h1F8-2-2 antibody administration test on human CX3CR1-KI mice (evaluation of CX3CR1-expressing cells in blood)

Human CX3CR1-KI female mice at 8 or 9 weeks of age were untreated or intraperitoneally administered with 200 µg per mouse of the h1F8-2-2 antibody, BII00313 or the Synagis antibody, and after 1, 3, 5 and 7 days, peripheral blood (heparin whole blood) was collected from the abdominal aorta of each mouse. In 96-well plates, the peripheral blood was dispensed in 100 µL volumes, and a detection antibody mixture was added and reacted at 4°C for 20 minutes. After addition of 1 × RBC Lysis/Fixation (Biolegend), the blood was suspended and allowed to stand at room temperature for 20 minutes, whereby erythrocytes were hemolyzed and the stained cells were fixed. After the cells were washed several times with 1% FBS/PBS, FACSLyric and FlowJo (BD Biosciences) were used for analysis.

As detection antibodies, a labeled anti-CD45 antibody (BD Biosciences), an anti-CD11b antibody (Biolegend) and an anti-CD115 antibody (Biolegend) were used. In addition, for use as anti-hCX3CR1 antibodies, two antibodies with different epitopes (m1F8-2 antibody and 66B02-mFc) were labeled with Alexa 488.

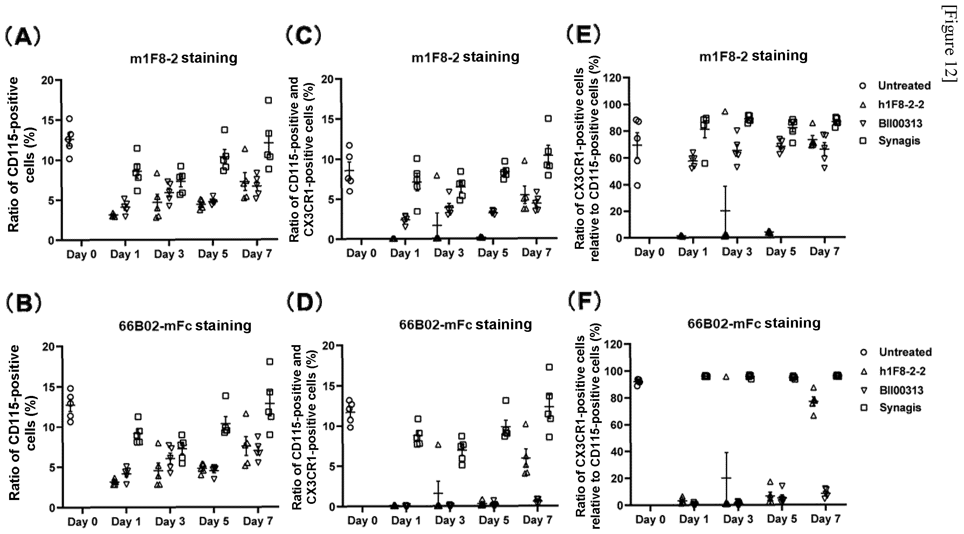

The labeled cells were developed on forward scatter and side scatter light, and a cell population depleted of cell debris and cell aggregates (Singlet) was set to 100%. The ratio of CD45, CD11b and CD115-positive cells in Singlet cells is shown in Figure 12(A) and (B) (A: m1F8-2 staining, B: 66B02-mFc staining). The h1F8-2-2 antibody-receiving group and the BII00313-receiving group showed a 50% or more reduction in CD115 positive cells on Day 1 after administration, and then showed slow recovery over time until Day 7.

The ratio of CD45, CD11b, CD115 and CX3CR1-positive cells in Singlet cells is shown in Figure 12 (C) and (D) (C: m1F8-2 staining, D: 66B02-mFc staining). In the h1F8-2-2 antibody-receiving group, m1F8-2 positive and 66B02-mFc positive cells were not substantially observed until Day 5 after administration. On the other hand, in the BII00313-receiving group, 66B02-mFc positive cells were not substantially observed until Day 7 after administration, whereas m1F8-2 positive cells were observed on Day 1 after administration.

Further, the ratio of CX3CR1 positive cells relative to CD115 positive cells which were set to 100% is shown in Figure 12(E) and (F) (E: m1F8-2 staining, F: 66B02-mFc staining). In the h1F8-2-2 antibody-receiving group, m1F8-2 positive cells or 66B02-mFc positive cells on CD115 positive cells were not substantially observed until Day 5 after administration. On the other hand, in the BII00313-receiving group, 66B02-mFc positive cells on CD115 positive cells were not substantially observed until Day 7 after administration, whereas m1F8-2 antibody positive cells were observed on Day 1 after administration.

The foregoing results indicate that the h1F8-2-2 antibody and BII00313 both have the effect of reducing CD115 positive cells. In addition, the foregoing results suggest differences in their properties because the h1F8-2-2 antibody has the function of attenuating CX3CR1 (receptor) per se on the CD115 positive cell membrane, whereas BII00313 has the function of occupying the receptor, but its function of attenuating CX3CR1 is weaker than that of the h1F8-2-2 antibody.

### [Example 16]

### 16. Antibody administration test in human CX3CR1-KI mouse late-onset EAE model

### 16-1. Evaluation of the number of cells infiltrated into the central nervous system upon m1F8-2 antibody administration

NR4A2-cKO/human CX3CR1-KI mice induced to develop monophasic EAE in the same manner as shown in Example 7 were administered with 200 µg of m1F8-2 or an isotype negative antibody thereof (mIgG2a) on Day 24 and Day 31 after EAE induction. In the same manner as shown in 9-2 in Example 9, on Day 32 after EAE induction, the brain and spinal cord were taken from each mouse to evaluate the number of cells infiltrated into the central nervous system. Samples from 3 mice of each antibody-receiving group were pooled and used. In the same manner as shown in 9-2 in Example 9, the cells were enriched, stimulated, stained, fixed and analyzed. Among CD45, TCRβ and CD4-positive cells, the ratio of Eomes and IFN-y double-positive cells (A) and the ratio of Eomes and Granzyme B (GZMB) double-positive cells (B) are shown in Table 12. For separation and detection of the cells, the following antibodies were used: an anti-CD45 antibody (Biolegend), an anti-TCRβ antibody (Biolegend), an anti-CD4 antibody (Biolegend), an anti-IFN-y antibody (Biolegend), an anti-Eomes antibody (eBioscience), and an anti-Granzyme B antibody (Biolegend).

As shown in Table 12A, the m1F8-2 antibody-receiving group showed a decrease in Eomes positive cells (upper) or Eomes and IFN-y double-positive cells (lower). In addition, as shown in Table 12B, the m1F8-2 antibody-receiving group showed a decrease in Eomes positive cells (upper) or Eomes and GZMB double-positive cells (lower). CD4, Eomes and IFN-γ-positive or CD4, Eomes and GZMB-positive cells are interpreted to mean cytotoxic Th cells, and a series of results suggested a possibility that the m1F8-2 antibody would act on CX3CR1 to reduce cytotoxic Th cells in the central nervous system, i.e., would inhibit or suppress the infiltration of cytotoxic Th cells into the CNS, thereby resulting in strongly improved EAE pathology.

### [Table 12]

**Table 12**

| (A) | | |
|---|---|---|
| Antibody administered | Negative antibody | m1F8-2 |
| Ratio of Eomes positive cells (%) | 7.88 | 4.42 |
| Ratio of Eomes and IFN-γ double-positive cells (%) | 5.64 | 2.67 |

| (B) | | |
|---|---|---|
| Antibody administered | Negative antibody | m1F8-2 |
| Ratio of Eomes positive cells (%) | 14.1 | 4.97 |
| Ratio of Eomes and GZMB double-positive cells (%) | 0.78 | 0.24 |

### 16-2. Evaluation of EAE score upon h1F8-2-2 antibody administration

NR4A2-cKO/human CX3CR1-KI mice induced to develop monophasic EAE in the same manner as shown in Example 7 were administered with 200 µg of h1F8-2-2 or an isotype antibody thereof (Synagis) on Day 24 and Day 28 after EAE induction, and EAE pathology was evaluated for each mouse.

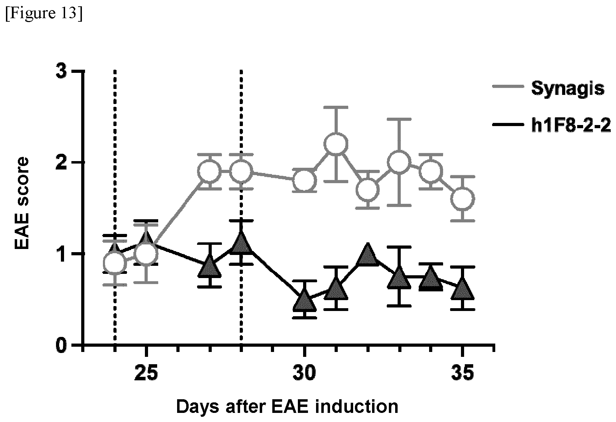

As shown in Figure 13, the h1F8-2-2 antibody-receiving group showed strongly improved EAE pathology.

### 16-3. Evaluation of the number of cells infiltrated into the central nervous system upon h1F8-2-2 antibody administration

NR4A2-cKO/human CX3CR1-KI mice induced to develop monophasic EAE in the same manner as shown in Example 7 were administered with 200 µg of h1F8-2-2 or an isotype negative antibody thereof (Synagis) on Day 24 and Day 28 after EAE induction. In the same manner as shown in 16-1 above, on Day 31 after EAE induction, the brain and spinal cord were taken from each mouse to evaluate the number of cells infiltrated into the central nervous system. Samples from 7 mice of the negative control antibody-receiving group or samples from 5 mice of the h1F8-2-2 antibody-receiving group were pooled and used. In the same manner as shown in 16-1 above, the cells were enriched, stimulated, stained, fixed and analyzed. Among CD45, TCRβ and CD4-positive cells, the ratio of CX3CR1 positive cells, the ratio of Eomes positive cells, and the ratio of Eomes and CX3CR1 double-positive cells are shown in Table 13. For separation and detection of the cells, the following antibodies were used: an anti-CD45 antibody (Biolegend), an anti-TCRβ antibody (Biolegend), an anti-CD4 antibody (Biolegend), an anti-Eomes antibody (eBioscience), and an anti-CX3CR1 antibody (Biolegend).

As shown in Table 13, the h1F8-2-2 antibody-receiving group showed a decrease in CX3CR1 positive cells (upper), Eomes positive cells (middle) or Eomes and CX3CR1 double-positive cells (lower). A series of results suggested a possibility that as in the case of the m1F8-2 antibody, the h1F8-2-2 antibody would act on CX3CR1 to reduce cytotoxic Th cells in the central nervous system, i.e., would inhibit or suppress the infiltration of cytotoxic Th cells into the CNS, thereby resulting in strongly improved EAE pathology.

### [Table 13]

**Table 13**

| Antibody administered | Synagis | h1F8-2-2 |
|---|---|---|
| Ratio of CX3CR1 positive cells (%) | 10.5 | 6.05 |
| Ratio of Eomes positive cells (%) | 7.57 | 1.27 |
| Ratio of Eomes and CX3CR1 double-positive cells (%) | 0.51 | 0.10 |

### [Example 17]

### 17. Antibody administration test in human CX3CR1-KI mouse CAIA (anti-collagen antibody cocktail-induced arthritis) model

### 17-1. CAIA model induction

Human CX3CR1-KI male mice at 7 weeks of age were intraperitoneally administered with 5 mg per mouse of an anti-collagen antibody cocktail (Chondrex). On Day 3 after antibody cocktail administration, the mice were intraperitoneally administered with 50 µg per mouse of LPS (Chondrex) to induce a CAIA model. The untreated group (Untreated) was administered with physiological saline, instead of the cocktail and LPS.

### 17-2. Evaluation of arthritis score upon m1F8-2 antibody administration

The human CX3CR1-KI mice administered with the anti-collagen antibody cocktail were intraperitoneally administered with 400 µg of the m1F8-2 antibody or an isotype negative antibody thereof, mIgG2a, on Day 3 and Day 10 after antibody cocktail administration. As a positive control drug, dexamethasone (Dex, SIGMA) was orally administered at 6 µg daily on Day 3 to Day 14 after antibody cocktail administration. All the animals were measured daily for the arthritis scores of four limbs on Day 1 to Day 14 after antibody cocktail administration. In accordance with the following evaluation criteria, scores were observed and recorded for three joints of finger, back and wrist in each limb, and the total score of four limbs was shown.

### <Evaluation criteria for arthritis>

Score 0: normal (no inflammation in all joints)
Score 1: mild but visible redness and swelling in the ankle or wrist, or limited visible redness and swelling in each finger (inflammation in any one joint)
Score 2: moderate redness and swelling in the ankle or wrist (inflammation in any two joints)
Score 3: severe redness and swelling in the whole paw including fingers (inflammation in all joints)
Score 4: severe inflammation in limbs including multiple joints (inflammation in all joints and red swelling in the whole limb)

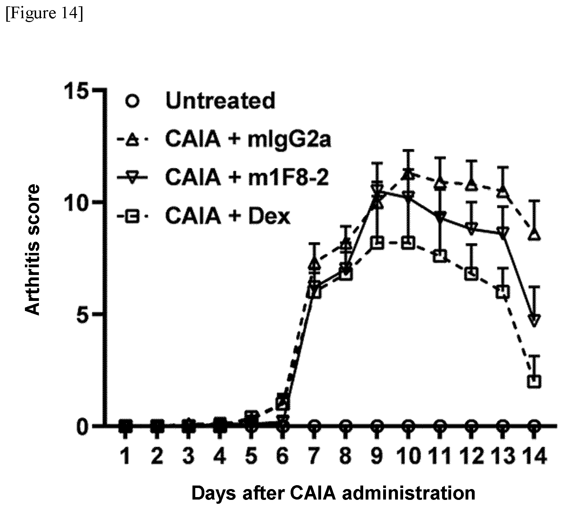

As shown in Figure 14, the CAIA-receiving groups showed increased arthritis scores in human CX3CR1-KI mice. When compared to the group receiving negative control mIgG2a, the group receiving positive control dexamethasone was confirmed to have a strong alleviating effect on arthritis. Likewise, the m1F8-2-receiving group was also confirmed to have an alleviating effect on arthritis.

### Industrial Applicability

The present invention enables the provision of an antibody binding to human CX3CR1, etc. The anti-human CX3CR1 antibody according to the present invention may have, for example, inhibitory or suppressive activity against the interaction or binding between human CX3CR1 and human CX3CL1 (fractalkine), or the function of improving experimental autoimmune encephalomyelitis (EAE) pathology or suppressing the progression of EAE pathology. Moreover, the anti-human CX3CR1 antibody according to the present invention may exert an effect as a prophylactic agent, a progression suppressor or a therapeutic agent for progressive immune-mediated demyelinating diseases (preferably progressive immune-mediated demyelinating diseases of the central nervous system, e.g., secondary progressive multiple sclerosis (SPMS)).

### Sequence Listing Free Text

SEQ ID NO: 1: synthetic peptide
SEQ ID NO: 3: synthetic peptide
SEQ ID NO: 4: synthetic peptide
SEQ ID NO: 5: synthetic peptide
SEQ ID NO: 6: synthetic peptide
SEQ ID NO: 9: synthetic peptide
SEQ ID NO: 10: synthetic peptide
SEQ ID NO: 11: synthetic peptide
SEQ ID NO: 12: synthetic peptide
SEQ ID NO: 13: synthetic peptide
SEQ ID NO: 14: synthetic peptide
SEQ ID NO: 15: synthetic peptide

## Claims

1. An antibody against human CX3CR1, wherein
the amino acid sequences of heavy chain variable region (VH) complementarity determining region (CDR) 1 (CDR-H1), CDR2 (CDR-H2) and CDR3 (CDR-H3) consist of:
the amino acid sequences shown in SEQ ID NOs: 2, 3 and 4, respectively, and
the amino acid sequences of light chain variable region (VL) CDR1 (CDR-L1), CDR2 (CDR-L2) and CDR3 (CDR-L3) consist of:
the amino acid sequences shown in SEQ ID NOs: 6, 7 and 8, respectively; or
the amino acid sequences shown in SEQ ID NOs: 10, 7 and 8, respectively.

2. The antibody according to claim 1, wherein
(a)
the amino acid sequences of CDR-H1, CDR-H2 and CDR-H3 consist of the amino acid sequences shown in SEQ ID NOs: 2, 3 and 4, respectively, and
the amino acid sequences of CDR-L1, CDR-L2 and CDR-L3 consist of the amino acid sequences shown in SEQ ID NOs: 6, 7 and 8, respectively, or
(b)
the amino acid sequences of CDR-H1, CDR-H2 and CDR-H3 consist of the amino acid sequences shown in SEQ ID NOs: 2, 3 and 4, respectively, and
the amino acid sequences of CDR-L1, CDR-L2 and CDR-L3 consist of the amino acid sequences shown in SEQ ID NOs: 10, 7 and 8, respectively.

3. An antibody against human CX3CR1, wherein
the amino acid sequence of the heavy chain variable region (VH) consists of the amino acid sequence shown in SEQ ID NO: 1, 11, 12, 13 or 14, and
the amino acid sequence of the light chain variable region (VL) consists of the amino acid sequence shown in SEQ ID NO: 5, 9 or 15.

4. The antibody according to claim 3, wherein
(a)
the amino acid sequence of VH consists of the amino acid sequence shown in SEQ ID NO: 1, and
the amino acid sequence of VL consists of the amino acid sequence shown in SEQ ID NO: 5,
(b)
the amino acid sequence of VH consists of the amino acid sequence shown in SEQ ID NO: 1, and
the amino acid sequence of VL consists of the amino acid sequence shown in SEQ ID NO: 9,
(c)
the amino acid sequence of VH consists of the amino acid sequence shown in SEQ ID NO: 11, and
the amino acid sequence of VL consists of the amino acid sequence shown in SEQ ID NO: 15,
(d)
the amino acid sequence of VH consists of the amino acid sequence shown in SEQ ID NO: 12, and
the amino acid sequence of VL consists of the amino acid sequence shown in SEQ ID NO: 15,
(e)
the amino acid sequence of VH consists of the amino acid sequence shown in SEQ ID NO: 13, and
the amino acid sequence of VL consists of the amino acid sequence shown in SEQ ID NO: 15, or
(f)
the amino acid sequence of VH consists of the amino acid sequence shown in SEQ ID NO: 14, and
the amino acid sequence of VL consists of the amino acid sequence shown in SEQ ID NO: 15.

5. The antibody according to any one of claims 1 to 4, wherein the antibody is a humanized antibody.

6. The antibody according to any one of claims 1 to 4, which has inhibitory or suppressive activity against the interaction or binding between human CX3CR1 and human CX3CL1 (fractalkine).

7. The antibody according to any one of claims 1 to 4, which has inhibitory or suppressive activity against the function of Eomes-expressing Th cells.

8. The antibody according to claim 7, which has inhibitory or suppressive activity against the infiltration of Eomes-expressing Th cells into the CNS.

9. The antibody according to any one of claims 1 to 4, which has the function of improving experimental autoimmune encephalomyelitis (EAE) pathology or suppressing the progression of EAE pathology.

10. The antibody according to any one of claims 1 to 4, which is used for the prevention, suppression of progression, or treatment of a progressive immune-mediated demyelinating disease, preferably a progressive immune-mediated demyelinating disease of the central nervous system; an autoimmune disease, preferably rheumatoid arthritis, inflammatory bowel disease, Crohn's disease, systemic scleroderma, systemic lupus erythematosus, or autoimmune myositis; a heart disease including atherosclerosis; a chronic renal disease; a diabetic heart disease; a diabetic renal disease; an interstitial lung disease; a cancer, preferably breast cancer, prostate cancer, or colorectal cancer; a COVID-19-related central nervous system disease; or a neurodegenerative disease, preferably ALS, Alzheimer's disease, or multiple system atrophy.

11. The antibody according to claim 10, wherein the progressive immune-mediated demyelinating disease is secondary progressive multiple sclerosis (SPMS).

12. An antibody fragment derived from the antibody according to any one of claims 1 to 4.

13. A pharmaceutical composition comprising the antibody according to any one of claims 1 to 4 or the antibody fragment according to claim 12.

14. The pharmaceutical composition according to claim 13, which is used for the prevention, suppression of progression, or treatment of a progressive immune-mediated demyelinating disease, preferably a progressive immune-mediated demyelinating disease of the central nervous system; an autoimmune disease, preferably rheumatoid arthritis, inflammatory bowel disease, Crohn's disease, systemic scleroderma, systemic lupus erythematosus, or autoimmune myositis; a heart disease including atherosclerosis; a chronic renal disease; a diabetic heart disease; a diabetic renal disease; an interstitial lung disease; a cancer, preferably breast cancer, prostate cancer, or colorectal cancer; a COVID-19-related central nervous system disease; or a neurodegenerative disease, preferably ALS, Alzheimer's disease, or multiple system atrophy.

15. The pharmaceutical composition according to claim 14, wherein the progressive immune-mediated demyelinating disease is secondary progressive multiple sclerosis (SPMS).

16. The pharmaceutical composition according to claim 13, which is used to inhibit or suppress the interaction or binding between human CX3CR1 and human CX3CL1 (fractalkine).

17. The pharmaceutical composition according to claim 13, which is used to inhibit or suppress the function of Eomes-expressing Th cells.

18. The pharmaceutical composition according to claim 17, which is used to inhibit or suppress the infiltration of Eomes-expressing Th cells into the CNS.

19. The pharmaceutical composition according to claim 13, which is used to improve experimental autoimmune encephalomyelitis (EAE) pathology or suppress the progression of EAE pathology.

20. A method for the prevention, suppression of progression, or treatment of a progressive immune-mediated demyelinating disease, preferably a progressive immune-mediated demyelinating disease of the central nervous system; an autoimmune disease, preferably rheumatoid arthritis, inflammatory bowel disease, Crohn's disease, systemic scleroderma, systemic lupus erythematosus, or autoimmune myositis; a heart disease including atherosclerosis; a chronic renal disease; a diabetic heart disease; a diabetic renal disease; an interstitial lung disease; a cancer, preferably breast cancer, prostate cancer, or colorectal cancer; a COVID-19-related central nervous system disease; or a neurodegenerative disease, preferably ALS, Alzheimer's disease, or multiple system atrophy, wherein the method comprises administering the pharmaceutical composition according to claim 13 to a subject.

21. The method according to claim 20, wherein the progressive immune-mediated demyelinating disease is secondary progressive multiple sclerosis (SPMS).

22. A method for inhibiting or suppressing the interaction or binding between human CX3CR1 and human CX3CL1 (fractalkine), wherein the method comprises administering the pharmaceutical composition according to claim 13 to a subject.

23. A method for inhibiting or suppressing the function of Eomes-expressing Th cells, wherein the method comprises administering the pharmaceutical composition according to claim 13 to a subject.

24. The method according to claim 23, which is a method for inhibiting or suppressing the infiltration of Eomes-expressing Th cells into the CNS, wherein the method comprises administering the pharmaceutical composition according to claim 13 to a subject.

25. A method for improving experimental autoimmune encephalomyelitis (EAE) pathology or suppressing the progression of EAE pathology, wherein the method comprises administering the pharmaceutical composition according to claim 13 to a subject.

26. A kit for use in the prevention, suppression of progression, or treatment of, or for use in the diagnosis of a progressive immune-mediated demyelinating disease, preferably a progressive immune-mediated demyelinating disease of the central nervous system; an autoimmune disease, preferably rheumatoid arthritis, inflammatory bowel disease, Crohn's disease, systemic scleroderma, systemic lupus erythematosus, or autoimmune myositis; a heart disease including atherosclerosis; a chronic renal disease; a diabetic heart disease; a diabetic renal disease; an interstitial lung disease; a cancer, preferably breast cancer, prostate cancer, or colorectal cancer; a COVID-19-related central nervous system disease; or a neurodegenerative disease, preferably ALS, Alzheimer's disease, or multiple system atrophy, wherein the kit comprises the antibody according to any one of claims 1 to 4, or the antibody fragment according to claim 12.

27. The kit according to claim 26, wherein the progressive immune-mediated demyelinating disease is secondary progressive multiple sclerosis (SPMS).
